# EUROPEAN PATENT APPLICATION

(11) **EP 2 796 152 A1**
(43) Date of publication of application: **29.10.2014**
(21) Application number: 13165281.0
(22) Date of filing: 25.04.2013
(51) Int. Cl.: A61K 49/00, C07F 7/00

(54) **Unsymmetrical Bis Azainositol Hafnium Complexes for X-Ray Imaging**

(71) Applicant: Bayer Pharma Aktiengesellschaft, 13353 Berlin (DE)
(72) Inventor: Berger, Markus, 13347 Berlin (DE); Suelzle, Detlev, 13465 Berlin (DE); Frenzel, Thomas, 12247 Berlin (DE); Graham, Keith, 10405 Berlin (DE); Jost, Gregor, 10318 Berlin (DE); Neis, Christian, 66123 Saarbrücken (DE); Hegetschweiler, Kaspar, 66123 Saarbrücken (DE)
(74) Representative: BIP Patents

(57) **Abstract**

The present invention describes a new class of trinuclear hafnium complexes comprising two hexadentate azainositol carboxylic acid ligands, methods for their preparation and their use as X-ray contrast agents.

## Description

### Field of the invention

The present invention relates to new bis azainositol hafnium complexes comprising new azainositol ligands, to methods of preparing said compounds and to the use of said compounds as X-ray contrast agents.

### Background of the invention

The synthesis and co-ordination chemistry of 1,3,5-triamino-1,3,5-trideoxy-*cis*-inositol (taci) and a multitude of derivatives of this cyclohexane-based polyamino-polyalcohol have widely been examined in the past by Hegetschweiler et al. (Chem. Soc. Rev. 1999, 28, 239). Among other things, the ability of taci and of the hexa-*N,N',N"-*methylated ligand tdci to form trinuclear complexes of the composition [M₃(H-₃taci)₂]³⁺ and [M₃(H-₃tdci)₂]³⁺, respectively, with a unique, sandwich-type cage structure in the presence of heavy metals M^{III} like Bi^{III} or a series of lanthanides was described (Chem. Soc. Rev. 1999, 28, 239; Inorg. Chem. 1993, 32, 2699; Inorg. Chem. 1998, 37, 6698). But, due to their moderate solubility in water and their deficient thermodynamic stability, these complexes proved not to be suitable for *in vivo* applications.

Complex formation of taci with more than 30 metal ions has been investigated and the metal cations can be divided into five categories according to the adopted coordination mode that was verified by crystal structure analyses *(*Chem. Soc. Rev. 1999, 28, 239). Although this classification helpfully reviews the coordination properties of taci, it has to be pointed out that multiple metals do not fit into the presented scheme. As a consequence, a prediction of the preferred coordination mode for metals that have not been described so far is often ambiguous. In addition to that, it was demonstrated that modifications at the ligand backbone can have a strong impact on the coordination behavior *(*Inorg. Chem. 1997, 36, 4121). This is not only reflected in the structural characteristics of the metal complexes but can often lead to unpredictable changes in their thermodynamic and/or kinetic complex stability, water solubility and other physicochemical parameters. The ability to form trinuclear hafnium complexes with a sandwich-type cage structure has never been reported before for any taci derivative.

Moreover, the synthesis of mononuclear carboxylic acid derived taci metal complexes has been reported by Laboratorien Hausmann AG, St. Gallen, CH in DE 40 28 139 A1 and WO 92/04056 A1 for iron^{III} and gadolinium^{III}. A possible application of its mononuclear, radioactive metal complexes as radiopharmaceuticals was also claimed.

All-*cis*-1,3,5-triamino-2,4,6-cyclohexane triol derivatives, their use and methods for their preparation were also described by Laboratorien Hausmann AG in EP, A, 190 676.

Byk Gulden Lomberg Chemische Fabrik GmbH described taci based transition metal complexes for magnetic resonance diagnostics in WO 91/10454.

Nycomed AS in WO 90/08138 described heterocyclic chelating agents for the preparation of diagnostic and therapeutic agents for magnetic resonance imaging, scintigraphy, ultrasound imaging, radiotherapy and heavy metal detoxification.

The formation of trinuclear iron^{III} complexes was suggested by G. Welti (Dissertation, Zürich 1998**)** for an acetate and by A. Egli (Dissertation, Zürich 1994**)** for a 2-hydroxybenzyl derivative of taci. G. Welti also described the synthesis of rhenium^{V} and rheniums^{VII} complexes of acetate derived ligands based on taci with a M₁L₁ stoichiometry.

D. P. Taylor & G. R. Choppin (Inorg. Chim. Acta 2007, 360, 3712) described the formation of mononuclear lanthanide complexes with similar derived ligands and determined a pM value of 6.0 for complexes with europium^{III} which means that the stability under physiological conditions is even lower than that of europium^{III} complexes of unmodified taci.

The WO2013/00970 A1 described a new class of highly stable Tungsten complexes, so W₃O₂ Clusters and their use as X-ray contrast agents.

General Electric Company described nanoparticle compositions in WO 2012/080290 comprising metal oxides including hafnium oxide and their potential use as contrast agents in medical imaging techniques such as X-ray. This relates to an earlier report on dextrin-stabilized aquasols of zirconium and hafnium dioxides (Zirconotrast, Hafnotrast) in the context of their biokinetics *(*Environmental Research 1979, 18, 127).

Since the iodine content of iodinated CT contrast agents that are administrated today is 45 % or even higher, polynuclear metal complexes are needed to significantly improve the attenuation properties. Mononuclear metal complexes like (NMG)₂GdDTPA (Janon E. A. Am. J. Roentgen 1989, 152, 1348) or YbDTPA (Unger E., Gutierrez F. Invest. Radiol. 1986, 21, 802) proved to be well-tolerated alternatives for patients that are contraindicated for iodinated agents but a reduction in the radiation doses and/or the contrast agent dosages can only be achieved when the metal content is comparable to the content of iodine in the current X-ray contrast agents. All compounds described above in or out of the context with diagnostic applications hold either only one metal center bound to the complex and the metal content of ≤ 30 % is significantly lower than 40% or the present metal is, not suited for a X-ray CT application due to its low absorption coefficient, e.g. iron.

Hafnium is characterized by a higher absorption coefficient for X-rays than iodine, especially in the range of tube voltages normally used in modern CT. A modern CT X-ray-tube, however, requires a minimum voltage of about 70 kV and reaches maximum voltage of 160 kV. As future technical developments in CT will not substantially change these parameters, iodine generally does not provide ideal attenuation features for this technology. In comparison to iodine the attenuation optimum (k-edge) of hafnium corresponds better to the ranges of voltages used in CT. Therefore the new hafnium complexes require a similar or lower contrast media dosage than conventional triiodinated contrast agents.

The use of hafnium based contrast agents will allow more flexibility for CT scanning protocols and lead to scan protocols that provide equivalent diagnostic value at lower radiation doses. Especially this feature is of high importance for CT. As technical development goals in terms of spatial and temporal resolution have approached the limit of clinical significance, reduction of the radiation burden of CT scanning has today become a central aspect of the development of new CT scanners and X-ray machines. Following the widely accepted ALARA-rule (radiation exposure has to be reduced to levels: As Low As Reasonably Achievable), the new hafnium based contrast agents will contribute to high-quality diagnostic imaging at reduced radiation exposure.

In summary, the state of the art described above consists of either physiologically stable heavy metal complexes with a low metal content per molecule or complexes with a high metal content, which are not thermodynamically stable enough for a physiological application or hold a metal that is not suitable for a diagnostic X-ray CT application.

The aim of the present invention was to provide sufficiently stable, water soluble and well tolerated hafnium complexes with a high metal content for use as X-ray contrast agents in diagnostic imaging, especially in modern computed tomography.

This aim was achieved by the provision of the compounds of the present invention. It has been found, that tri-*N*,*N'*,*N"-*substituted derivatives of taci (L) effectively form new complexes with hafnium of a M₃L₂ stoichiometry which grants a high metal content of > 35% for the compounds of the present invention. Surprisingly, it was observed that these complexes show a very high stability in aqueous solution for this type of stoichiometry under heat sterilization conditions and have an excellent tolerability in experimental animals as well as a high *in vivo* stability.

After intravenous injection the compounds of the present invention are excreted fast and quantitatively via the kidneys, comparable to the well established triiodinated X-ray contrast agents.

The invention of suitable new bis-azainositol hafnium complexes enables for the first time the practical use of this compound class as X-ray contrast agents in diagnostic imaging.

By enabling and developing novel hafnium-based contrast agents a clear advantage over the existing iodine-based contrast agents is offered as the radiative dose for the higher absorption coefficient of hafnium-based contrast agents is significantly reduced in comparison to the iodine-based contrast agents.

### Summary of the invention

The present invention describes a new class of trinuclear hafnium complexes comprising two hexadentate azainositol carboxylic acid ligands, methods for their preparation and their use as X-ray contrast agents.

### Detailed description of the invention

In a first aspect, the invention is directed to compounds of the general formula (I), 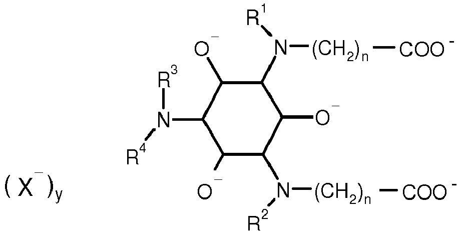 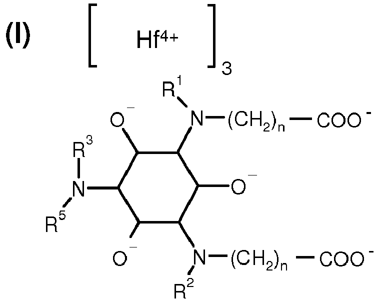 wherein
the substituents at the cyclohexyl ring exhibit an all-*cis* configuration;
R¹, R² and R³ are independently from each other H or CH₃;
R⁴ is H, CH₃, CH₂CH₃, CH₂CH₂CH₃, CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), CH(CH₂OH)(CH₂COO⁻), CH(CH₂CH₂OH)(COO⁻), or CH(CH₂OH)(COO⁻);
R⁵ is H, CH₃, CH₂CH₃, CH₂CH₂CH₃, CH₂CH₂OH, CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH), CH(CH₂OH)(CH₂COO⁻), CH(CH₂CH₂OH)(COO⁻), or CH(CH₂OH)(COO⁻);
n is 1 or 2;
m is 1 or 2;
y is 0, 1 or 2;
   and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

The present invention includes all possible stereoisomers of the compounds of the present invention, as single stereoisomers, or as any mixture of said stereoisomers, e.g. R- or S- isomers, in any ratio. Isolation of a single stereoisomer, e.g. a single enantiomer or a single diastereomer, of a compound of the present invention may be achieved by any suitable state of the art method, such as chromatography, especially chiral chromatography, for example. Compounds containing chiral centers may be used as racemic mixture or as an enantiomerically enriched mixture or as a diastereomeric mixture or as a diastereomerically enriched mixture, and an individual stereoisomer may be used alone.

The present invention also relates to useful forms of the compounds as disclosed herein, such as metabolites, hydrates, solvates, prodrugs, salts, in particular pharmaceutically acceptable salts, and co-precipitates.

Trinuclear hafnium complexes of the general formula (I), which are charged at physiological pH, can be neutralized by addition of suitable, physiologically biocompatible counter ions.

Suitable anions are the anions of inorganic acids, such as, for example, hydrochloric acid, phosphoric acid and sulfuric acid, as well as the anions of organic acids, such as, for example, acetic acid, citric acid, aspartic acid, glutamic acid, among others can be used.

The compounds of the present invention can exist in the form of a salt. Said salt may be any salt, either an organic or inorganic salt, particularly any pharmaceutically acceptable organic or inorganic salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

Pharmaceutically acceptable salts of the compounds according to the invention include salts of mineral acids, carboxylic acids and sulfonic acids, for example salts of hydrochloric acid, sulfuric acid, phosphoric acid, methanesulfonic acid, ethanesulfonic acid, toluenesulfonic acid, benzenesulfonic acid, acetic acid, propionic acid, lactic acid, tartaric acid, malic acid, citric acid, fumaric acid, maleic acid and benzoic acid.

The present invention includes all possible salts of the compounds of the present invention as single salts, or as any mixture of said salts, in any ratio.

For the manufacture of diagnostic agents, for example the administration to human or animal subjects, the compounds of general formula (I) will conveniently be formulated together with pharmaceutical carriers or excipient. The contrast media of the invention may conveniently contain pharmaceutical formulation aids, for example stabilizers, antioxidants, pH adjusting agents, flavors, and the like. They may be formulated for parenteral or enteral administration or for direct administration into body cavities. For example, parenteral formulations contain a sterile solution or suspension in a concentration range from 150 to 600 mg Hafnium/mL, especially 200 to 450 mg Hafnium/mL of the new azainositol heavy metal clusters according to this invention. Thus the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen and oxygen, such as ²H (deuterium), ³H (tritium), ¹¹C, ¹³C, ¹⁴C, ¹⁵N, ¹⁷O and ¹⁸O, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

Where the plural form of the word compounds, salts, polymorphs, hydrates, solvates and the like, is used herein, this is taken to mean also a single compound, salt, polymorph, isomer, hydrate, solvate or the like.

In accordance with a second embodiment of the first aspect, the present invention covers compounds of general formula (I), *supra,* in wherein :
the substituents at the cyclohexyl ring exhibit an all-*cis* configuration;
R¹, R² and R³ are independently from each other H or CH₃;
R⁴ is H, CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), CH(CH₂OH)(CH₂COO⁻), CH(CH₂CH₂OH)(COO⁻), or CH(CH₂OH)(COO⁻);
R⁵ is H, CH₂CH₂OH, CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH), CH(CH₂OH)(CH₂COO⁻), CH(CH₂CH₂OH)(COO⁻), or CH(CH₂OH)(COO⁻);
n is 1 or 2;
m is 1 or 2;
y is 0, 1 or 2;
   and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

In accordance with a third embodiment of the first aspect, the present invention covers compounds of general formula (I), *supra,* wherein :
the substituents at the cyclohexyl ring exhibit an all-*cis* configuration;
R¹, R² and R³ are independently from each other H or CH₃;
R⁴ is CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), or CH(CH₂CH₂OH)(COO⁻);
R⁵ is H, CH₂CH₂OH, CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH), CH(CH₂OH)(CH₂COO⁻), CH(CH₂CH₂OH)(COO⁻), or CH(CH₂OH)(COO⁻);
n is 1 or 2;
m is 1 or 2;
y is 0, 1 or 2;
   and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

In accordance with a fourth embodiment of the first aspect, the present invention covers compounds of general formula (I), *supra,* wherin :
the substituents at the cyclohexyl ring exhibit an all-*cis* configuration;
R¹, R² and R³ are independently from each other H or CH₃;
R⁴ is CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), or CH(CH₂CH₂OH)(COO⁻);
R⁵ is H, CH₂CH₂OH, CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH), or CH(CH₂CH₂OH)(COO⁻);
n is 1 or 2;
m is 1 or 2;
y is 0, 1 or 2;
   and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

Another embodiment of the first aspect are compounds of formula (I) selected from the group consisting of:
[Hf₃(H₋₃macitp)(H₋₃macidp)OH] = Hydroxido-3κ*O*-[µ₃-3,3',3"-({[1*R*-(1α,2α,3α,4α,5α,6α)]-2,4,6-trihydroxylato-1κ²*O*²*O⁶*,2κ²*O*²*O*⁴,3κ²*O*⁴*O*⁶-cyclohexane-1,3,5-triyl}tris{methylimino-1κ*N*¹,2κ*N*³,3κ*N*⁵])tripropanoato-1κ*O*,2κ*O*',3κ*O*"] [µ₃-3,3'-({[1*R*-(1α,2α,3α,4α,5α,6α)]-2,4,6-trihydroxylato-1 κ²*O*²*O⁶*,2κ²*O*²*O*⁴,3κ²*O*⁴O⁶-5-[methylamino-3κ*N*⁵]cyclohexane-1,3-diyl}bis-{methylimino-1 κ*N*¹,2κ*N*³})dipropanoato-1κ*O*,2κ*O*']trihafnium(IV) , 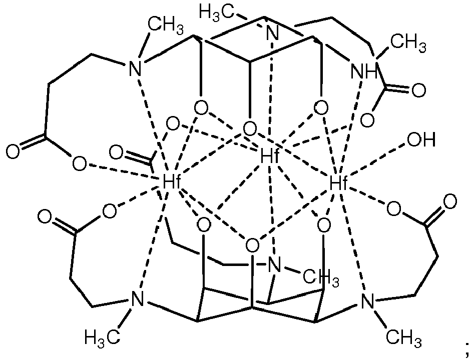
[Hf₃(H₋₄tacidadhp)₂] = Bis[µ₃-2,2'-({[1*R*-(1α,2α,3α,4α,5α,6α)]-2,4,6-trihydroxylato-1 κ²*O*²*O*⁶,2κ²*O*²O⁴,3κ²*O*⁴*O*⁶-5-[(3-hydroxy-2-hydroxylato-3KO-propyl)amino-3κ*N⁵*]cyclohexane-1,3-diyl]diimino-1κ*N*¹,2κ*N*³)diacetato-1κ*O*,2κ*O'*]trihafnium (IV), 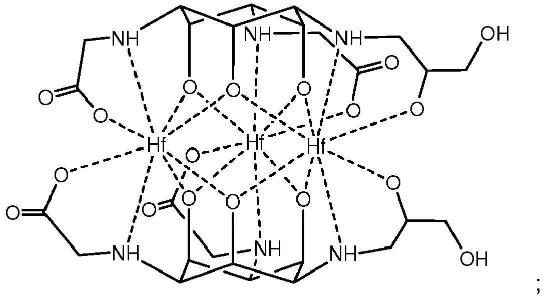
[Hf₃(H-₄tacidphe)₂] = Bis[µ₃-3,3'-({[1*R*-(1α,2α,3α,4α,5α,6α)]-2,4,6-trihydroxylato-1 κ²*O*²*O*⁶,2κ²*O*²*O*⁴,3κ²*O*⁴O⁶-5-[(2-hydroxylato-3κ*O*-ethyl)amino-3κ*N*⁵]cyclohexane-1,3-diyl}diimino-1κ*N*¹,2κ*N*³)dipropanoato-1κ*O*,2k*O*']trihafnium(IV) , 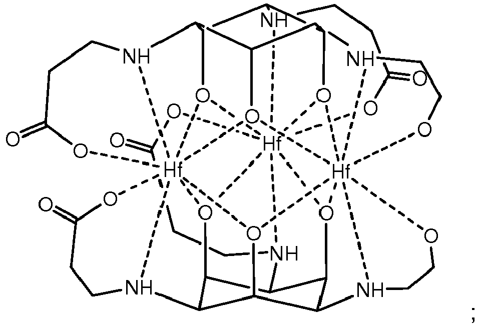
[Hf₃(H-₄tacidpdhp)₂] = Bis[µ₃-3,3'-({[1*R*-(1α,2α,3α,4α,5α,6α)]-2,4,6-trihydroxylato-1 κ²*O*²O⁶,2κ²*O*²O⁴,3κ²*O*⁴*O*⁶-5-[(3-hydroxy-2-hydroxylato-3κ*O*-propyl)amino-3κ*N⁵*]cyclohexane-1,3-diyl]diimino-1κ*N*¹,2κ*N*³)dipropanoato-1κ*O*,2κ*O*']trihafnium(IV) , 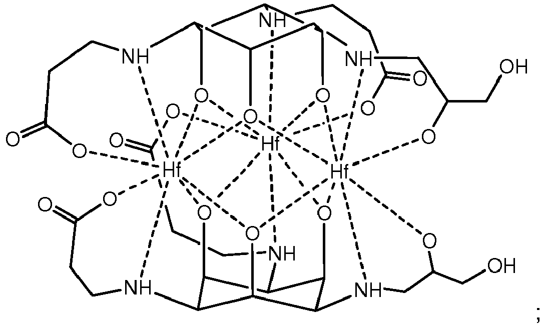
[Hf₃(H-₄tacidpery)₂] = Bis[µ₃-3,3'-({[1*R*-(1α,2α,3α,4α,5α,6α)]-5-[(2*S*,3*R*)(3,4-dihydroxy-2-hydroxylato-3κ*O*-butyl)amino-3κ*N*⁵]-2,4,6-trihydroxylato-1 κ²*O*²*O*⁶,2κ²*O*²*O*⁴,3κ²*O*⁴O⁶-cyclohexane-1,3-diyl]diimino-1κ*N*¹,2κ*N*³)dipropanoato-1κ*O*,2κ*O*']trihafnium(IV) , 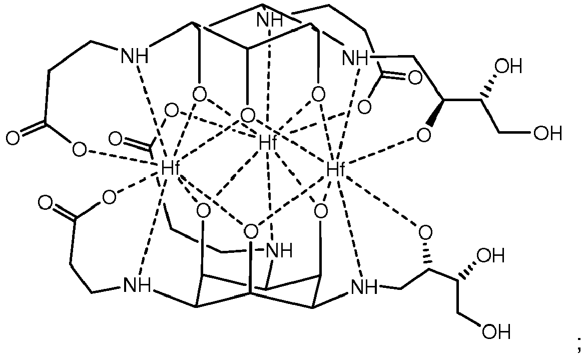
[Hf₃(H-₄tacidaery)₂] = Bis[µ₃-2,2'-({[1*R*-(1α,2α,3α,4α,5α,6α)]-5-[(2*S*,3*R*)(3,4-dihydroxy-2-hydroxylato-3K*O*-butyl)amino-3κ*N⁵*]-2,4,6-trihydroxylato-1κ²*O*²*O⁶*,2κ²*O*²*O*⁴,3κ²*O*⁴O⁶-cyclohexane-1,3-diyl]diimino-1κ*N*¹,2κ*N*³)diacetato-1κ*O*,2κ*O*']trihafnium(IV) , 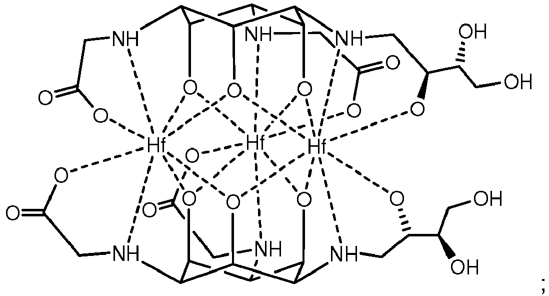
[Hf₃(H-₃tacidpma)₂] = Bis[µ₃-3,3'-({[1*R*-(1α,2α,3α,4α,5α,6α)]-5-[(carboxylato-3κ*O*-methyl)-amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)dipropanoato-1k*O*,2κ*O'*]trihafnium(IV), 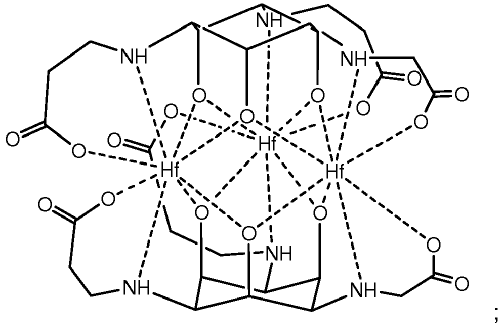
[Hf₃(H-₄tacidahe)₂] = Bis[µ₃-2,2'-({[1*R*-(1α,2α,3α,4α,5α,6α)]-2,4,6-trihydroxylato-1 κ²*O*²O⁶,2κ²*O*²*O*⁴,3κ²*O*⁴*O*⁶-5-[(2-hydroxylato-3κ*O*-ethyl)amino-3κ*N*⁵]cyclohexane-1,3-diyl}-diimino-1κ*N*¹,2κ*N*³)diacetato-1κ*O*,2κ*O*']trihafnium(IV) , 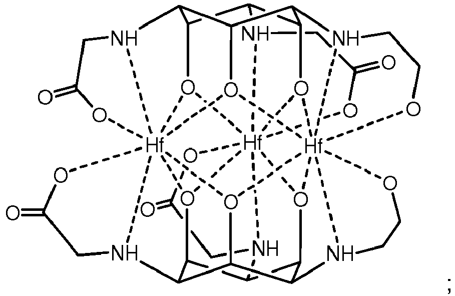
[Hf₃(H₋₄tacidahp)₂] = Bis[µ₃-2,2'-({[1*R*-(1α,2α,3α,4α,5α,6α)]-2,4,6-trihydroxylato-1 κ²*O*²*O*⁶,2κ²*O*²O⁴,3κ²*O*⁴*O*⁶-5-[(3-hydroxylato-3κ*O*-propyl)amino-3κ*N*⁵]cyclohexane-1,3-diyl}diimino-1κ*N*¹,2κ*N*³)diacetato-1κ*O*,2κ*O'*]trihafnium(IV), 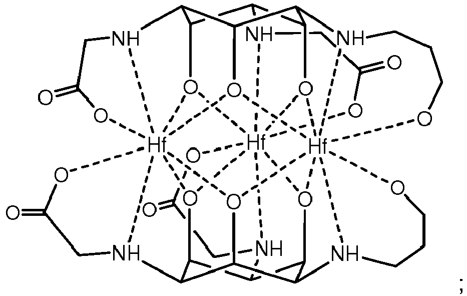
[Hf₃(H₋₃tacidamp)₂] = Bis[µ₃-2,2'-({[1*R*-(1α,2α,3α,4α,5α,6α)]-5-[(2-carboxylato-3κ*O*-ethyl)-amino-3κ*N*⁵]-2,4,6-trihydroxylato-1κ²*O*²O⁶,2κ²*O*²*O*⁴,3κ²*O*²O⁶-cyclohexane-1,3-diyl}diimino-1κ*N*¹,2κ*N*³)diacetato-1κ*O*,2κ*O*']trihafnium(IV), 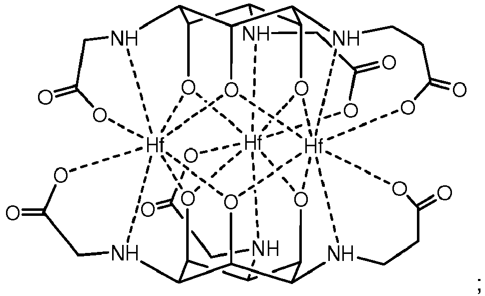
[Hf₃(H₋₄tacidadha)₂] = Bis[µ₃-2,2'-({[1*R*-(1α,2α,3α,4α,5α,6α)]-2,4,6-trihydroxylato-1 κ²*O*²O⁶,2κ²*O*²*O*⁴,3κ²*O*²*O⁶*-5-[(1-hydroxy-3-hydroxylato-3κ*O*-propan-2-yl)amino-3κ*N*⁵]-cyclohexane-1,3-diyl}diimino-1κ*N*¹,2κ*N*³)diacetato-1κ*O*,2κ*O*]trihafnium(IV), 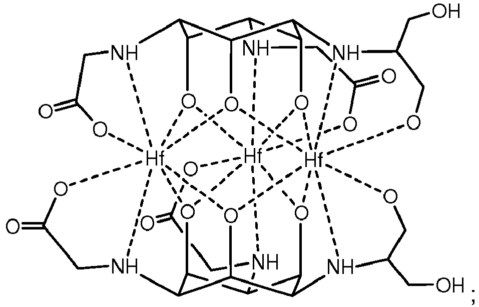
[Hf₃(H-₄tacidaethru)₂] = Bis[µ₃-2,2'-({[1*R*-(1α,2α,3α,4α,5α,6α)]-5-[(3,4-dihydroxy-1-hydroxylato-3κ*O*-butan-2-yl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)diacetato-1κ*O*,2κ*O*']trihafnium(IV), 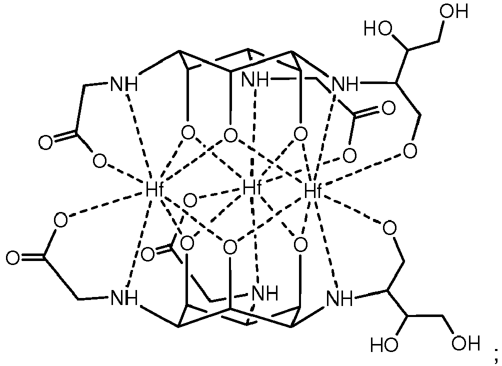 and
[Hf₃(H-₄tacidaghb)₂] = Bis[µ₃-2,2'-({[1*R*-(1α,2α,3α,4α,5α,6α)]-5-[(1-carboxylato-3κ*O*-3-hydroxypropan-1-yl)amino-3κ*N*⁵]-2,4,6-trihydroxylato-1κ²*O*²O⁶,2κ²*O*²O⁴,3κ²*O*²O⁶-cyclohexane-1,3-diyl]diimino-1κ*N*¹,2κ*N*³)diacetato-1κ*O*,2κ*O*']trihafnium(IV) ,

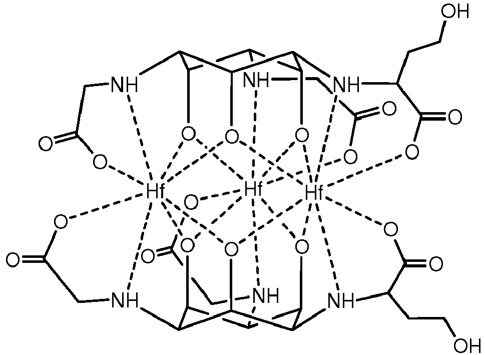

In a second aspect, the invention is directed to the process for the preparation of the compounds of the general formula (I).

In a third aspect, the invention is directed to the process for the preparation of the compounds of the general formula (I) from carboxylic acids of the general formula (II), 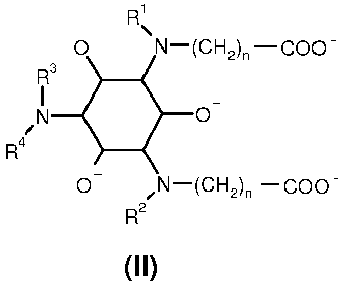 wherein
the substituents at the cyclohexyl ring exhibit an all-*cis* configuration;
R¹, R² and R³ are independently from each other H or CH₃;
R⁴ is H, CH₃, CH₂CH₃, CH₂CH₂CH₃, CH₂CH₂OH, CH₂CH₂CH₂OH, CH₂CH(OH)CH₂OH, CH₂CH(OH)CH(OH)CH₂OH, CH(CH₂OH)₂, (CH₂)mCOO⁻, CH(CH₂OH)(CH(OH)CH₂OH), CH(CH₂OH)(CH₂COO⁻), CH(CH₂CH₂OH)(COO⁻), or CH(CH₂OH)(COO⁻);
n is 1 or 2; and
m is 1 or 2;
and a metal (IV) halogenide,
wherein
metal is Hafnium;
   and
halogenide is either chloride or bromide,
and hydrates thereof,
in aqueous solution under elevated temperatures, using conventional methods or microwave irradiation, ranging from 80°C to 180°C, in a pH range of 1 to 7, preferably at 110 ° to 160 °C in a pH range of 2 to 7.

In an fourth aspect, the invention is directed to compounds of general formula (I) for the manufacture of diagnostic agents, especially of X-ray diagnostic agents for administration to humans or animals.

Another aspect of the invention is the use of the compounds of general formula (I) or mixtures thereof for the manufacture of diagnostic agents.

Another aspect of the invention is the use of a compound of general formula (I) for diagnostic imaging.

### General synthesis of compounds of the invention

The present invention provides carboxylic acid derived ligands based on 1,3,5-triamino-1,3,5-trideoxy-*cis*-inositol (taci) that can readily form trinuclear, highly stable hafnium(IV) complexes with high water solubility. The tri-O-benzylated taci derivative all-*cis*-2,4,6-tris(benzyloxy)-1,3,5-cyclohexanetriamine (tbca) was used as starting material throughout. It can be prepared as reported by Bartholoma et al. (Chem. Eur. J. 2010, 16 3326). The ligand tbca can be alkylated in the presence of bases like cesium carbonate or N,N-diisopropylethylamine with *tert*-butyl-halogenoacetate in organic solvents like THF or dichloromethane. The statistically formed monoalkylation **(tbcama)** and dialkylation (tbcada) products are be purified by preparative HPLC or by chromatography on amino phase silica gel. Depending on the equivalents of alkylating agent used, the twofold derived ligand tbcada or the one fold derived ligand tbcama (Scheme 1) can be obtained as main product.

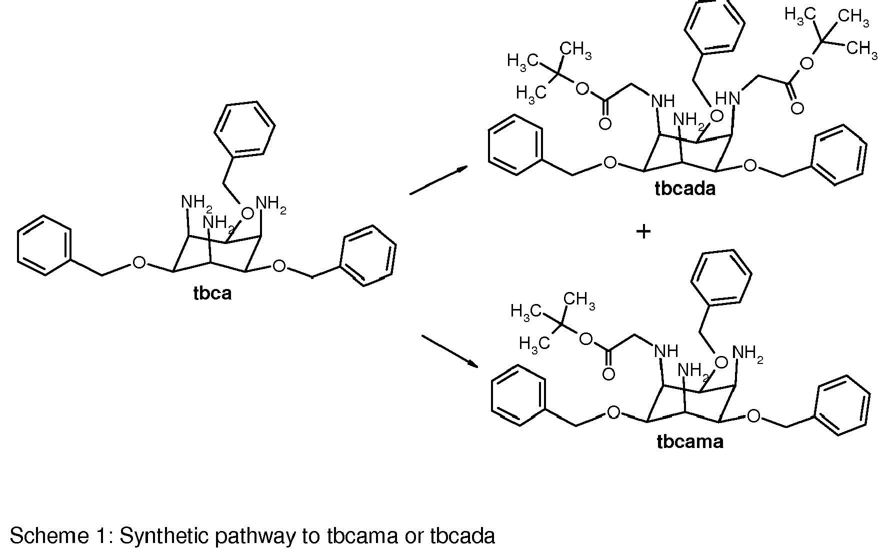

Introduction of the third amino substituent at the primary amine can be accomplished by reductive amination procedure or Michael addition of acrylic acid derivatives like acrylonitrile or acrylic esters. Aldehydes or ketones can be used in combination with a suitable reducing agent like 5-ethyl-2-methylpyridine borane complex *(*Tetrahedron Lett. 2008, 49, 5152), sodium triacetoxyborohydride or sodium cyanoborohydride *(*Comprehensive Organic Synthesis, Pergamon: Oxford 1991, 8, 25). In case of glycolaldehyde and D,L-glyceraldehyde the available dimers can be used to deliver the subsequent **tbcadamx** derivatives (Scheme 2). A third possibility to introduce a third substituent at the primary amine is the alkylation with halogenoalkanes in the presence of bases. The pure ligands are conveniently obtained in the hydrochloride form by cation exchange chromatography after removal of the protecting groups under strong acidic conditions.

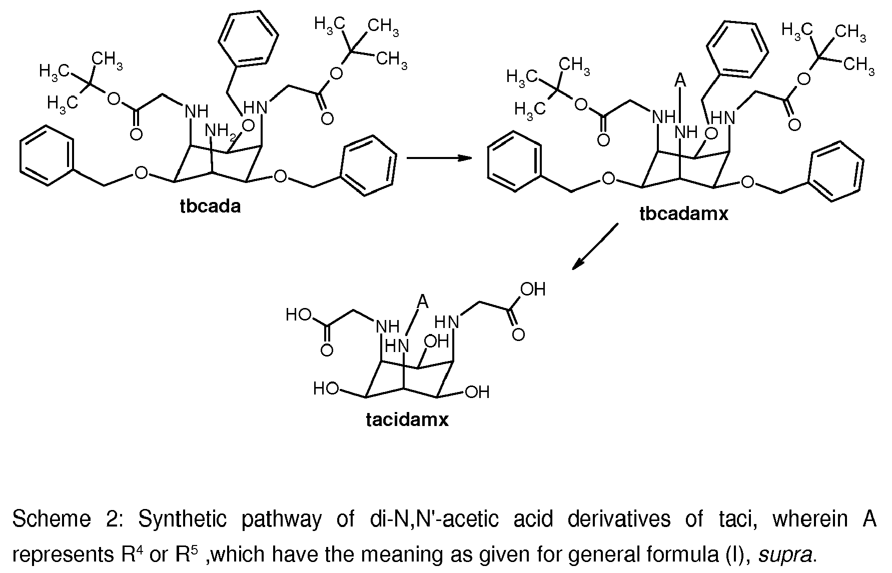

Starting from **tbcama** the ligand **tacidpma** with one acetic acid and two propionic acid as amino substituents can be prepared analogously by treatment with acrylonitrile or acrylic ester and subsequent acidic deprotection of the tbcadpma intermediate (Scheme 3).

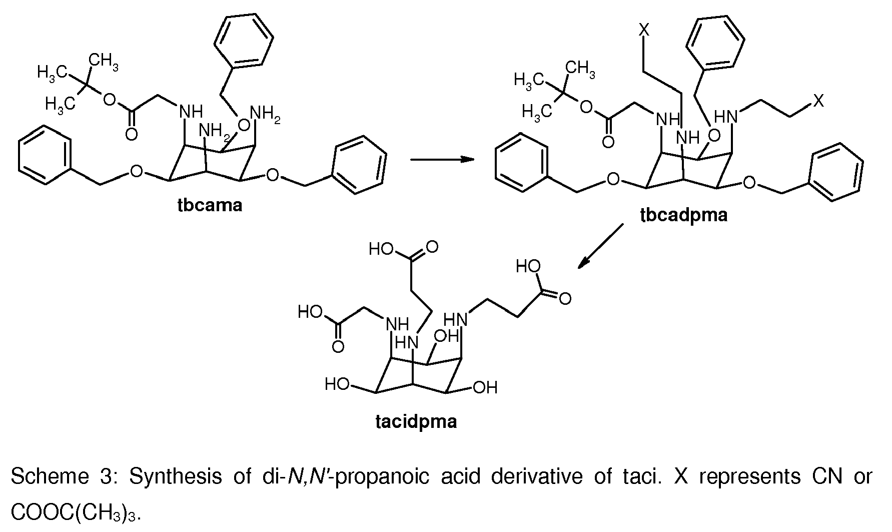

For the synthesis of further di-*N,N*'-propanoic acid derivatives of taci, the building block **tbcadpn** can be prepared from **tbca** using stoichiometric amounts of acrylonitrile. Introduction of the third amino substituent different from already present substituents to the intermediate **tbcadpmx** can analogously to **tbcadamx** be introduced at the primary amino group by reductive amination of appropriate aldehydes or ketones or by halogen-alkanes in the presence of base. (Scheme 4). The pure tacidpmx ligands can be obtained by simultaneous removal of the protecting groups and nitrile or ester hydrolysis under strong acidic conditions.

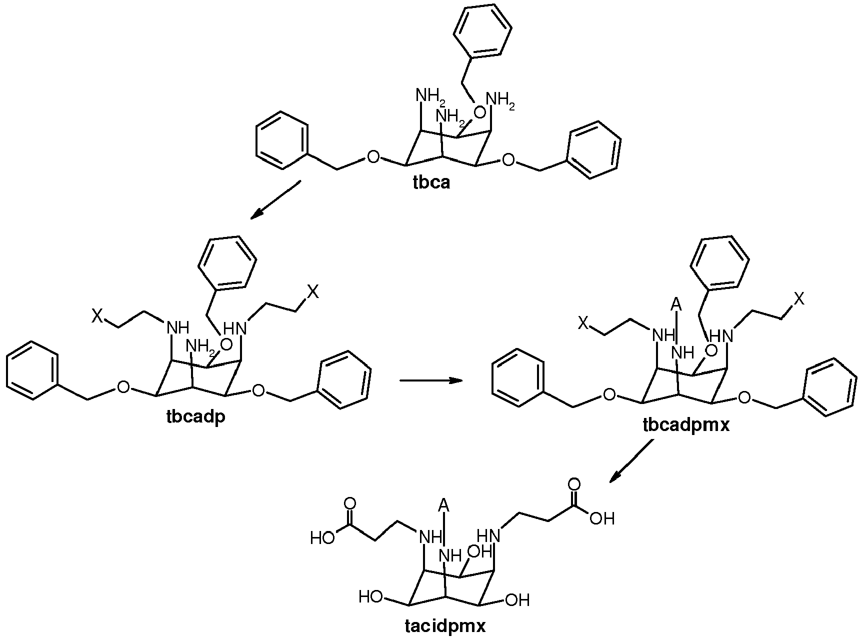

Scheme 4: Synthetic pathway of di-*N,N*'-propanoic acid derivatives of taci, wherein A represents R⁴ or R⁵, which have the meaning as given for general formula (I), *supra.* X represents CN or COOC(CH₃)₃.

Trinuclear hafnium(IV) complexes of the accordingly prepared ligands can be synthesized by adding stoichiometric amounts or minor excess of hafnium salts like hafnium(IV)chloride to aqueous solutions of the ligand (Scheme 5). The reaction mixtures can be heated by conventional methods or microwave irradiation at a pH range from 2 to 7 for at least 15 minutes at a temperature range from 110°C to 160°C under pressure.

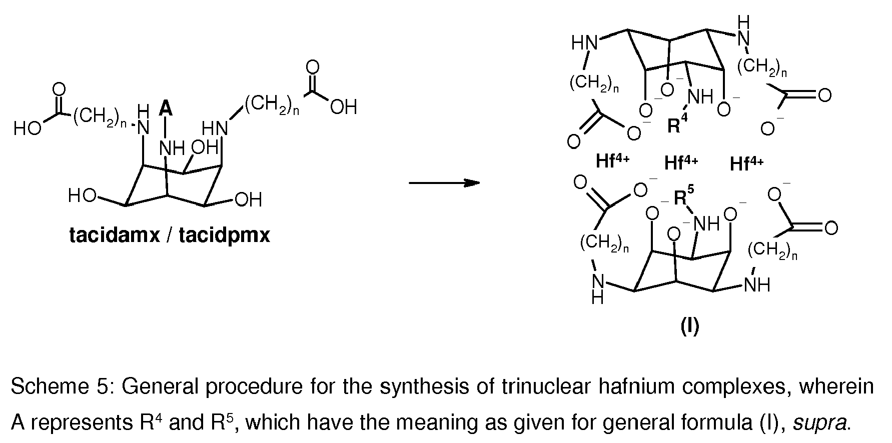

Isolation and purification of the desired complexes can be achieved by preparative HPLC, ultrafiltration or crystallization methods.

### Experimental Part

**Abbreviations**

| | |
|---|---|
| br | broad signal (in NMR data) |
| Cl | chemical ionisation |
| d | doublet |
| DAD | diode array detector |
| dd | doublet of doublet |
| ddd | doublet of doublet of doublet |
| dt | doublet of triplet |
| DMF | *N,N*-dimethylformamide |
| DMSO | dimethylsulfoxide |
| EI | electron ionisation |
| ELSD | evaporative light scattering detector |
| ESI | electrospray ionisation |
| EtOAc | ethyl acetate |
| Fmoc | fluorenylmethyloxycarbonyl |
| HPLC | high performance liquid chromatography |
| ICP-OES | Inductively coupled plasma - optical emission spectrometry |
| ICP-MS | Inductively coupled plasma - mass spectrometry |
| MeCN | acetonitrile |
| MS | mass spectrometry |
| m | multiplet |
| NH₄Cl | ammonium chloride |
| NMR | nuclear magnetic resonance spectroscopy: chemical shifts (δ) are given in ppm. |
| q | quadruplett (quartet) |
| rt | room temperature |
| *Rt* | retention time |
| s | singlet |
| t | triplet |
| THF | tetrahydrofuran |
| UPLC | ultra performance liquid chromatography |

### Materials and Instrumentation

The chemicals used for the synthetic work were of reagent grade quality and were used as obtained. Dowex 50 W-X2 (100-200 mesh, H+ form) was from Sigma-Aldrich, the mixed bed ion exchange resins Amberlite MB-6113 from Merck. The starting materials 1,3,5-triamino-1,3,5-trideoxy-*cis*-inositol (= taci) (Ghisletta M., Jalett H.-P., Gerfin T., Gramlich V., Hegetschweiler K. Helv. Chim. Acta 1992, 75, 2233) and all-*cis*-2,4,6-tris(benzyloxy)-1,3,5-cyclohexanetriamine (= tbca) (Bartholomä, M.; Gisbrecht, S.; Stucky, S.; Neis, C.; Morgenstern, B.; Hegetschweiler, K. Chem. Eur. J. 2010, 16, 3326) were prepared as described in the literature.

¹H and ¹³C{¹H} NMR spectra were measured in D₂O or DMSO-d₆, respectively (294 K, Bruker DRX Avance 400 MHz NMR spectrometer (*B*₀ = 9.40 T), resonance frequencies: 400.20 MHz for ¹H and 100.63 MHz for ¹³C or 300 MHz spectrometer for ¹H. Chemical shifts are given in ppm relative to sodium (trimethylsilyl)propionate-d₄ (D₂O or tetramethylsilane (DMSO-*d₆*) as internal standards (δ = 0 ppm). The pH* of the D₂O samples was adjusted using appropriate solutions of DCI in D₂O The term pH* refers to the direct pH-meter reading (Metrohm 713 pH meter) of the D₂O samples, using a Metrohm glass electrode with an aqueous (H₂O Ag/AgCl-reference that was calibrated with aqueous (H₂O) buffer solutions.

Elemental analyses (C,H,N) were recorded on a LECO 900V or VARIO EL analyzer.

Examples were analyzed and characterized by the following HPLC based analytical methods to determine characteristic retention time and mass spectrum:
**Method 1: UPLC** (ACN-HCOOH):
   Instrument: Waters Acquity UPLC-MS SQD 3001; column: Acquity UPLC BEH C18 1.7 50x2.1mm; eluent A: water + 0.1% formic acid, eluent B: acetonitril; gradient: 0-1.6 minutes 1-99% B, 1.6-2.0 minutes 99% B; flow 0.8 mL/minute; temperature: 60 °C; injection: 2 µL; DAD scan: 210-400 nm; ELSD
**Method 2: UPLC** (ACN-HCOOH polar):
   Instrument: Waters Acquity UPLC-MS SQD 3001; column: Acquity UPLC BEH C18 1.7 50x2.1mm; eluent A: water + 0.1% formic acid, eluent B: acetonitril; gradient: 0-1.7 minutes 1-45% B, 1.7-2.0 minutes 45-99% B; flow 0.8 mL/minute; temperature: 60 °C; injection: 2 µL; DAD scan: 210-400 nm; ELSD

### Examples

### Example 1 [Hf₃(H-₃macitp)(H-₃macidp)OH]

Hydroxido-3κ*O*-[µ₃-3,3',3"-({[1*R*(1α,2α,3α,4α,5α,6α)]-2,4,6-trihydroxylato-1 κ²*O*²*O*⁶, 2k²*O*²O⁴,3κ²*O*⁴O⁶-cyclohexane-1,3,5-triyl}tris{methylimino-1κ*N*¹,2κ*N*³,3κ*N*⁵}) tri-propanoato-1 κ*O*,2κ*O*',3κ*O*"] [µ₃-3,3'-({[1*R*(1α,2α,3α,4α,5α,6α)]-2,4,6-trihydroxylato-1κ²*O*²*O*⁶,2κ²*O*²*O*⁴,3κ²*O*⁴O⁶,5-[methylamino-3κ*N⁵*]cyclohexane-1,3-diyl}bis{methyl-imino-1 κ*N*¹,2κ*N*³})dipropanonato-1κ*O*,2κ*O*']trihafnium (IV)

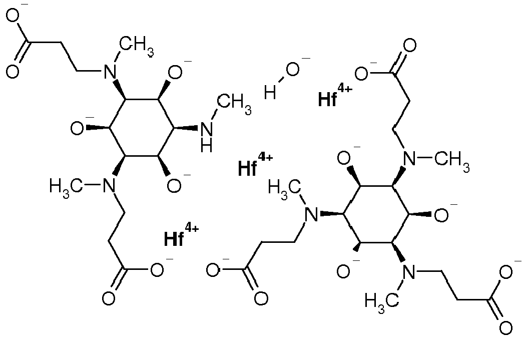

### Example 1 a

### 1,3,5-Triamino-1,3,5-trideoxy-cis-inositol-tri-N,N,N'-propionitrile (tacitpn)

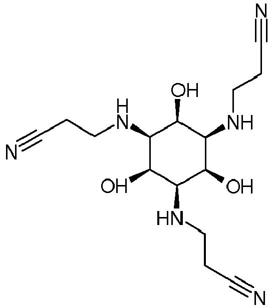

taci (2.0 g, 11.3 mmol) was dissolved in methanol (100 mL) and acrylonitrile (7.4 mL, 0.11 mol) was added. The solution was stirred for 24 hours at ambient temperature. The solvent was removed, the residue washed successively with diethyl ether and hexane and the white solid was dried in vacuo.
Yield: 3.9 g (97 %) tacitpn·0.2H₂O·0.5CH₃OH. Single crystals suitable for X-ray analysis were obtained by evaporation of a concentrated solution of tacitpn in methanol.

**¹H-NMR** (400 MHz, D₂O) δ = 2.72 (m, 9H), 3.03 (t, 6H), 4.23 (t, 3H) ppm.
**¹³C-NMR** (101 MHz, D₂O) δ = 20.5, 43.4, 60.1, 72.0, 123.2 ppm.
**Anal.** Calcd (%) for C₁₅H₂₄N₆O_{3·}0.2H₂O·0.5MeOH (356.01): C, 52.29; H, 7.47; N, 23.61. Found: C, 52.23; H, 7.23; N, 23.40.
**IR** (cm⁻¹): 602, 754, 843, 902, 1072, 1113, 1252, 1352, 1425, 1987, 2067, 2248, 2924, 3103, 3268.
**MS** (ES⁺): *mlz* (%) 337.5 (100) {tacitpn+H}⁺.
**MS** (ES⁻): *m*/*z*(%) 335.6 (100) {tacitpn-H}⁻.

### Example 1 b

### 1,3,5-Triamino-1,3,5-trideoxy-cis-inositol-tri-N,N',N"-propionic acid trihydrochloride (H₆tacitpCl₃)

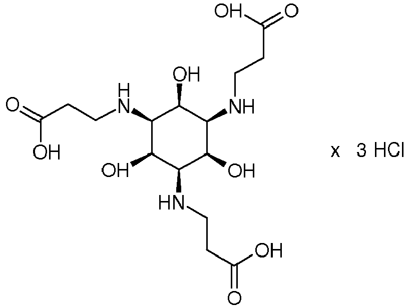

Tacitpn (3.8 g, 10.7 mmol) was dissolved in sodium hydroxide (10.3 g of a 25 % solution, 64.4 mmol) and heated to reflux for 4 h. The solvent was removed and the residue was taken up in 1 M hydrochloric acid (5 mL) and sorbed on DOWEX 50. The column was washed with water (1 L), 0.25 M hydrochloric acid (1 L), 1 M hydrochloric acid (1 L) and the product was eluted with 3 M hydrochloric acid (1 L). The solvent was removed and the solid dried in vacuo.
Yield: 5.1 g (86 %) H₃tacitp·3HCl·3H₂O.

**¹H-NMR** (400 MHz, D₂O) δ = 2.43 (t, 6H), 2.61 (m, 3H), 2.89 (t, 6H), 4.26 (m, 3H) ppm. **¹³C-NMR** (100 MHz, D₂O) δ = 40.3, 44.7, 60.5, 71.8, 184.2 ppm.
**Anal.** Calcd (%) for C₁₅H₂₇N₃O₉·3HCl·3H₂O (556.82): C, 32.36; H, 6.52; N, 7.55. Found: C, 32.56; H, 6.31; N, 7.64.
**MS** (ES⁺): *mlz* (%) 441.4 (100) {H₂tacitp+2Na}⁺, 394.2 (75) {H₃tacitp+H}⁺.
**MS** (ES-): *m*/*z*(%) 392.3 (100) {H₃tacitp-H}⁻.

### Example 1c

### 1,3,5-Trideoxy-1,3,5-tris(methylamino)-cis-inositol-tri-N,N',N"-propionic acid trihydrochlorid(H₆macitpCl₃)

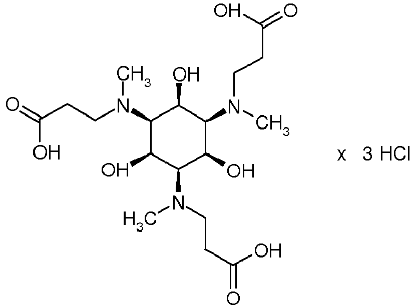

H₃tacitp·3HCl·3H₂O (400 mg, 0.7 mmol) was dissolved in a formaldehyde solution (37 %, 25 mL, 334 mmol) and palladium on charcoal (40 mg, 10 %) was added. The reaction mixture was hydrogenated in an autoclave at 50 atm H₂ for 4 days at rt. The reaction mixture was filtered off and the filtrate concentrated to dryness. The residue was dissolved twice in a 1 : 1 mixture of water and formic acid (30 mL) and evaporated to dryness again. The remaining solid was taken up in 3 M hydrochloric acid (10 mL) and sorbed on DOWEX 50. The column was washed successively with 0.5 M hydrochloric acid (1 L), 1 M hydrochloric acid (1 L) and 3 M hydrochloric acid (1 L). The 3 M fraction containing the product was evaporated to dryness and the solid was dried in vacuo.
Yield: 320 mg (71 %) H₃macitp·3HCl·4.5H₂O.

**¹H-NMR** (400 MHz, D₂O) δ = 3.04 (t, 6H), 3.15 (s, 9H), 3.67 (m, 3H), 3.78 (t, 6H), 5.04 (m, 3H) ppm.
**¹³C-NMR** (101 MHz, D₂O) δ = 23.6, 34.3, 45.5, 57.9, 58.6, 169.9 ppm.
**Anal.** Calcd (%) for C₁₈H₃₃N₃O₉·3HCl·4.5H₂O (625.92): C, 34.54; H, 7.25; N, 6.71. Found: C, 34.20; H, 6.86; N, 6.71.

### Example 1d

### Hydroxido-3κO-[µ₃-3,3',3"-({[1R-(1α,2α,3α,4α,5α,6α)]-2,4,6-trihydroxylato-1 κ²O²O⁶, 2κ²O²O⁴,3κ²O⁴O⁶-cyclohexane-1,3,5-triyl}tris{methylimino-1κN¹,2κN³,3κN⁵}) tri-propanoato-1κO,2κO',3κO"] [µ₃-3,3'-({[1R-(1α,2α,3α,4α,5α,6α)]-2,4,6-trihydroxylato-1 κ²O²O⁶,2κ²O²O⁴,3κ²O⁴O⁶-5-[methylamino-3κN⁵]cyclohexane-1,3-diyl}bis{methylimino-1κN¹,2κN³})dipropanonato-1κO,2κO']trihafnium (IV) [Hf₃(H₋₃macitp)(H₋₃macidp)OH]

A solution of 1,3,5-trideoxy-1,3,5-tris(methylamino)-*cis*-inositol-tri-*N,N',N*"-propionic acid trihydrochloride (900 mg, 1.49 mmol) and hafnium(IV)chloride (714 mg, 2.23 mmol) in water (16 mL) was separated into 3 pressure vessels. The pH of each vessel was adjusted to 4.5 by addition of aqueous sodium hydroxide (2 M) and water was added to reach a total volume of 30 mL. The vessels were sealed and irradiated in a microwave reactor for 20 minutes at 140°C. The combined solutions were treated with mixed bed ion exchange resins Amberlite MB-6113 until the resin kept its blue color. The filtrate was lyophilized, solved in water (300 mL) and passed through a 3000 da ultrafiltration membrane (Millipore YM3) while dilution of the retentate was repeated three times. The combined 3000 da filtrates were concentrated in vacuum while a final volume of 200 mL was lyophilized to yield 388 mg of raw product as a white solid which was purified by preparative HPLC to yield 122 mg of the title compound [Hf₃(H-₃macitp)(H-₃macidp)OH].

| | |
|---|---|
| *Column:* | C18 YMC-ODS AQ 10µm 51 x 200 mm |
| *Solvent:* | A = H₂O +0.1 % formic acid |
| | B = acetonitrile |
| *Gradient:* | 0 - 1 minute 1 % B, 1-10 minutes 1 - 25 % B |
| *Flow:* | 240 mL/minute |
| *Temperature:* | rt |
| *Detection:* | 195 nm |
| *Rt* in min: | 4.5 - 6.2 |

**¹H-NMR** (300 MHz, D₂O): δ = 2.37 - 2.58 (m, 10H), 2.59 - 2.93 (m, 27H), 3.05 (br., 1 H), 3.63 (br., 5H), 4.14 - 4.30 (m, 1 H), 4,80 (br, 1 H), 4.93 (br., 1 H), 5.20 (br., 1 H), 5.38 (br. , H) ppm.
**MS** (ES⁺): *m*/*z* (%)1323 (100) {[Hf₃(H₋₃macitp)(H₋₃macidp)]}⁺.

### Example 2 [Hf₃(H-₄tacidadhp)₂]

### Bis[µs-2,2'-({[1R(1α,2α,3α,4α,5α,6α)]-2,4,6-trihydroxylato-1 κ²O²O⁶,2κ²O²O⁴,3κ²O⁴O⁶-5-[(3-hydroxy-2-hydroxylato-3κO-propyl)amino-3κN⁵]cyclohexane-1,3-diyl}diimino-1 κN¹,2κN³)diacetato-1κO,2κO']trihafnium (IV)

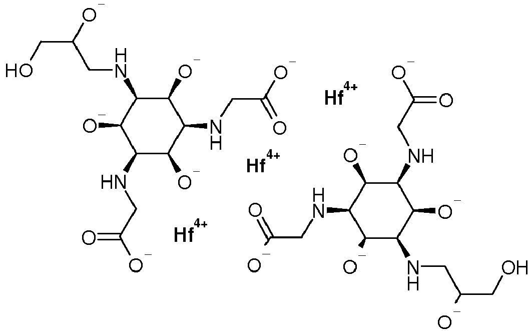

### Example 2a

### Di-tert-butyl 2,2'-({[1R(1α,2α,3α,4α,5α,6α)]-5-amino-2,4,6-tris[benzyloxy]cyclohexane-1,3-diyl}diimino)diacetate (tbcada)

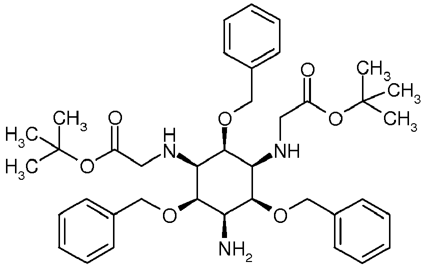

All-cis-2,4,6-tris(benzyloxy)-1,3,5-cyclohexanetriamine (2.0 g, 4.47 mmol, Chem. Eur. J., 2010, 16, 3326-3340) was dissolved in THF (72 mL) and cesium carbonate (1.60 g, 4.92 mmol) was added. *Tert*-butyl bromoacetate (1.66 g, 8.49 mmol) was added at room temperature and the mixture stirred for 20 hours. The mixture was filtered, the filtrate was concentrated under reduced pressure and the residue was purified by chromatography on amino phase silica gel (ethyl acetate in hexane, 40 to 100% then ethanol in ethyl acetate, 0 to 20%) to yield 0.76 g of the title compound.

**¹H-NMR** (300 MHz, DMSO-*d6*): δ = 1.38 (s, 18H), 2.71 (br. s., 2H), 3.26 - 3.35 (m, 6H), 3.39 - 3.43 (m, 2H), 3.50 - 3.59 (m, 2H), 4.49 - 4.69 (m, 6H), 7.20 - 7.45 (m, 15H) ppm.
**¹³C-NMR** (75 MHz, DMSO-*d6*): δ = 27.6, 51.4, 51.7, 57.1, 70.2, 70.3, 74.4, 75.7, 80.3, 127.3, 127.3, 127.5, 128.2, 137.9, 138.1, 171.8 ppm.
**MS** (ES⁺): *mlz* (%) 676 (27) {tbcada+H}⁺, 620 (100) {tbcada-^{t}Bu+H}⁺, 564 (48) {tbcada-2×^{t}Bu+H}+, 474 (8) {tbcada-2×^{t}Bu-Bn+H}⁺.

As a second product 0.95 g of *tert*-butyl 2-({[1*R*-(1α,2α,3α,4α,5α,6α)]-5-amino-2,4,6-tris[benzyloxy] cyclohexane-1,3-diyl}imino)acetate was isolated.

**¹H-NMR** (400 MHz, CDCl₃): δ = 1.47 (s, 9H), 3.08 (t, 1 H), 3.16 (t, 2H), 3.55 - 3.63 (m, 3H), 3.66 (s, 2H), 4.58 - 4.74 (m, 6H), 7.29 - 7.44 (m, 15H) ppm.

### Example 2b

### Di-tert butyl 2,2'-({[1R(1α,2α,3α,4α,5α,6α)]-2,4,6-tris[benzyloxy]-5-[(2,3-dihydroxypropyl)amino]cyclohexane-1,3-diyl}diimino)diacetate

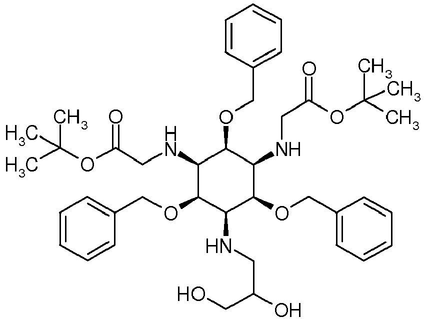

Tbcada (1.6 g, 2.4 mmol) was dissolved in methanol (150 mL). Acetic acid (500 µL), D,L-glyceraldehyde dimer (426 mg, 2.4 mmol) and 5-ethyl-2-methylpyridine borane (529 µL, 3.6 mmol) were successively added. The suspension which turned into a clear solution within few hours was stirred for 3 days at ambient temperature. The solvent was removed and the residue was purified via preparative HPLC (C18 column, solvent: water + 0.1 wt% formic acid (A) / acetonitrile (*B*); gradient: from 30 % *B* to 70 % *B* in 16 minutes; UV detection at 258 nm). The combined product fractions were lyophilized to yield 1.4 g of the title compond.

**¹H-NMR** (300 MHz, DMSO-*d6*): δ = 1.28 - 1.38 (m, 18H), 2.85 - 3.20 (m, 3H), 3.32 - 3.42 (m, 2H), 3.45 - 3.68 (m, 8H), 3.92 - 4.02 (m, 2H), 4.56 - 4.83 (m, 6H), 7.22 - 7.50 (m, 15H) ppm.

### Example 2c

### 2,2'-({[1R(1α,2α,3α,4α,5α,6α)]-2,4,6-Trihydroxy-5-[(2,3-dihydroxypropyl)amino]-cyclohexane-1,3-diyl}diimino)diacetic acid trihydrochloride (H₅tacidadpCl₃)

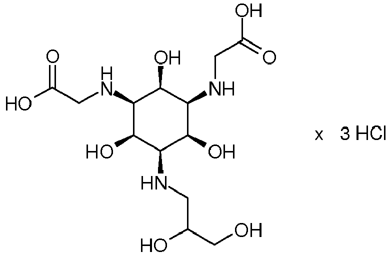

Di*-tert-*butyl 2,2'-({[1*R*-(1α,2α,3α,4α,5α,6α)]-2,4,6-tris[benzyloxy]-5-[(2,3-dihydroxypropyl)-amino]cyclohexane-1,3-diyl}diimino)diacetate (1.4 g) was suspended in hydrochloric acid (6 M, 150 mL) and heated to reflux for 5 h. After cooling, the solution was evaporated to dryness, the remaining solid dissolved in 0.5 M hydrochloric acid (5 mL) and sorbed on DOWEX 50. The column was washed with water (0.5 L) and 0.5 M hydrochloric acid (1 L) and the product was eluted with 3 M hydrochloric acid (1 L). The eluant was removed and the solid dried in vacuo to yield 852 mg of the title compound H₂tacidadp·3HCl·3H₂O.

**¹H-NMR** (400 MHz, D₂O, pH* = 0): δ = 3.30 (dd, 1 H), 3.50 (dd, 1 H), 3.67 (dd, 1 H), 3.73 (dd, 1 H), 3.80 (t, 1 H), 3.85 (m, 2H), 4.17 (m, 1 H), 4.23 (br, 4H), 4.78 (m, 3H) ppm.
**¹³C-NMR** (D₂O, pH* = 0): δ = 47.7, 50.3, 59.5, 59.6, 66.1, 66.2, 66.3, 69.9 171.1 ppm. **Anal.** Calcd (%) for C₁₃H₂₅N₃O₉·3HCl·3H₂O (530.78): C, 29.42; H, 6.46; N, 7.92. Found: C, 29.26; H, 6.12; N, 7.80.

### Example 2d

### Bis[µ₃-2,2'-({[1R(1α,2α,3α,4α,5α,6α)]-2,4,6-trihydroxylato-1 κ²O²O⁶,2κ²O²O⁴,3κ²O⁴O⁶-5-[(3-hydroxy-2-hydroxylato-3κO-propyl)amino-3κN⁵]cyclohexane-1,3-diyl}diimino-1 κN¹,2κN³)diacetato-1κO,2κO']trihafnium(IV) [Hf₃(H₋₄tacidadhp)₂]

A solution of H₂tacidadp·3HCl·3H₂O (1.4 g, 2.6 mmol) and hafnium(IV)chloride (1.44 g, 4.5 mmol) in water (100 mL) was separated into 10 pressure vessels. The pH of each vessel was adjusted to 7.0 by addition of aqueous ammonia (33%) and water was added to reach a total volume of 30 mL. The vessels were sealed and irradiated in a microwave reactor for 45 minutes at 140°C. After cooling the pH of the vessels was readjusted to 7.0 and irradiation in a microwave reactor at 140°C was continued for 3 hours. The reaction mixtures were combined and ultra-filtrated through a 500 da membrane while dilution of the retentate was repeated 3 times by addition of desalted water. The retentate was collected, diluted to a total volume of 1200 mL and passed through a 3000 da ultrafiltration membrane while dilution of the retentate was repeated two times. The combined 3000 da filtrates were concentrated in vacuum while a final volume of 200 mL was lyophilized and yielded 1.36 g of the title compound [Hf₃(H₋₃tacidadhp)₂].

**MS** (ES⁺): *mlz* (%) 1281.2 (100) {[Hf₃(H₋₃tacidadhp)₂]+Na}⁺.
**MS** (ES-): *mlz* (%) 1257.2 (100) {[Hf₃(H₋₃tacidadhp)₂]-H}⁻.

### Example 3 [Hf₃(H-₄tacidphe)₂]

### Bis[µ₃-3,3'-({[1R(1α,2α,3α,4α,5α,6α)]-2,4,6-trihydroxylato-1 κ²O²O⁶κ²O²O⁴,3κ²O⁴O⁶-5-[(2-hydroxylato-3κO-ethyl)amino-3κN⁵]cyclohexane-1,3-diyl}diimino-1κN¹,2κN³)dipropanoato-1 κO,2κO']trihafnium (IV)

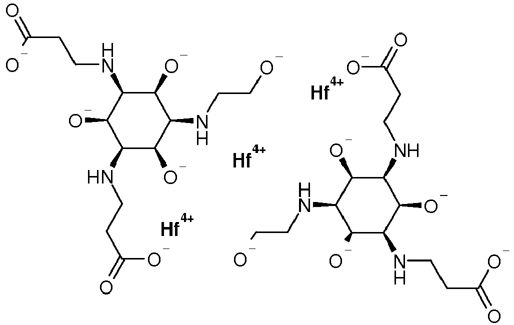

### Example 3a

### 3,3'-({[1R(1α,2α,3α,4α,5α,6α)]-5-Amino-2,4,6-tris[benzyloxy]cyclohexane-1,3-diyl}-diimino)dipropanenitrile (tbcadpn)

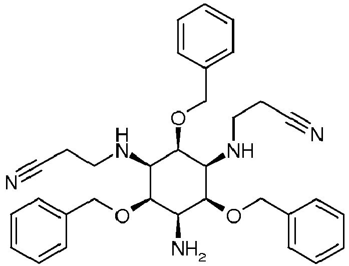

All-*cis*-2,4,6-tris(benzyloxy)-1,3,5-cyclohexanetriamine (1.0 g, 1.9 mmol) was dissolved in methanol (100 mL) and acrylonitrile (249 µL,3.8 mmol) was added. The solution was stirred for 3 days at ambient temperature. The solvent was removed and the residue purified via preparative HPLC (C18 column, solvent: water + 0.1 wt% formic acid (A) / acetonitrile (*B*); gradient: from 15 % *B* to 65 % *B* in 15 minutes; UV detection at 257 nm). The combined product fractions were lyophilized to yield 420 mg tbcadpn·HCOOH.

**¹H-NMR** (400 MHz, DMSO-*d6*): δ = 2.54 - 2.59 (m, 3H), 2.61 - 2.70 (m, 2H), 2.84 (m, 2H), 2.90 (m, 2H), 3.47 (m, 2H), 3.55 (m, 2H), 3.90 (m, 1 H), 4.56 (d, 2H), 4.59 (s, 2H), 4.68 (d, 2H), 7.24 - 7.43 (m, 15H), 8.32 (s, 1 H) ppm.

### Example 3b

### 3,3'-([[1R(1α,2α,3α,4α,5α,6α)]-2,4,6-Trihydroxy-5-[(2-hydroxyethyl)amino]cyclohexane-1,3-diyl}diimino)dipropanoic acid trihydrochloride (H₅tacidpheCl₃)

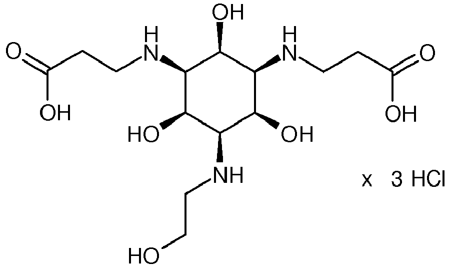

tbcadpn·HCOOH (200 mg, ~ 0.3 mmol) was dissolved in methanol (25 mL). Acetic acid (75 µL), glycolaldehyde dimer (50 mg, 0.4 mmol) and 5-ethyl-2-methylpyridine borane (80 µL, 0.5 mmol) were successively added. The clear solution was stirred for 3 days at ambient temperature. The solvent was removed and the residue purified via preparative HPLC (C18 column, solvent: water + 0.1 wt% formic acid (*A*) / acetonitrile (*B*); gradient: from 15 % *B* to 55 % *B* in 15 minutes; UV detection at 232 nm). The combined product fractions were lyophilized. The residue (50 mg) was suspended in 6 M hydrochloric acid (100 mL), heated to reflux for 3 hours and stirred for 12 hours at rt afterwards. The solution was evaporated to dryness. The remaining solid was dissolved in 0.5 M hydrochloric acid (5 mL) and sorbed on DOWEX 50. The column was washed with water (0.5 L) and 0.5 M hydrochloric acid (1 L) and the product was eluted with 3 M hydrochloric acid (1.5 L). The eluant was removed and the solid dried in vacuo to yield 40 mg H₂tacidphe·3HCl·xH₂O.

**¹H-NMR** (300 MHz, D₂O, pH* = 0): δ = 2.99 (t, 4H), 3.44 (m, 2H), 3.57 (t, 4H), 3.78 (m, 3H), 3.97 (m, 2H), 4.76 (m, 3H) ppm.

### Example 3c

### Bis[µ₃-3,3'-({[1R(1α,2α,3α,4α,5α,6α)]-2,4,6-trihydroxylato-1 κ²O²O⁶,2κ²O²O⁴,3κ²O⁴O⁶-5-[(2-hydroxylato-3κO-ethyl)amino-3kN⁵]cyclohexane-1,3-diyl}diimino-1κN¹,2κN³)dipropanoato-1 κO,2κO']trihafnium(IV) [Hf₃(H-₃tacidphe)₂]

H₂tacidphe·3HCl·xH₂O (35 mg, ~ 60 µmol) was dissolved in water (6 mL) and hafnium(IV)tetrachloride (30 mg, 94 µmol) dissolved in a small amount of water (2 mL) was added. The pH was adjusted to ~ 4.5 (1 M solution of sodium hydroxide) and the solution was irradiated 45 minutes at 140 °C in the microwave. A slight clouding was filtered off and the solution was desalted via ultrafiltration (cellulose acetate membrane, lowest NMWL 500 g/mol, Millipore). The retentate was again passed through an ultrafiltration cell (cellulose acetate membrane, lowest NMWL 3000 g/mol, Millipore). The filtrate was evaporated to dryness and the white solid dried in vacuo to yield: 29 mg of the title compound [Hf₃(H₋₃tacidphe)₂].

MS (ES-): *mlz* (%) 1300.3 (100) {[Hh(H₋₃tacidphe)₂]+HCOO}⁻.

### Example 4 [Hf₃(H₋₄tacidpdhp)₂]

### Bis[µ₃-3,3'-({[1R(1α,2α,3α,4α,5α,6α)]-2,4,6-trihydroxylato-1 κ²O²O⁶,2κ²O²O⁴,3κ²O⁴O⁶-5-[(3-hydroxy-2-hydroxylato-3κO-propyl)amino-3κN⁵]cyclohexane-1,3-diyl}diimino-1 κN¹,2κ) dipropanoato-1 κO,2κO']trihafnium(IV)

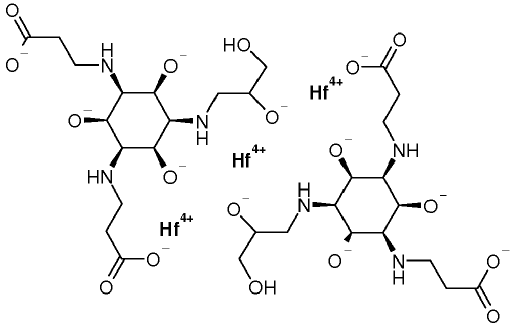

### Example 4a

### 3,3'-([[1R(1α,2α,3α,4α,5α,6α)]-5-[(2,3-Dihydroxypropyl)amino]-2,4,6-trihydroxy-cyclohexane-1,3-diyl}diimino)dipropanoic acid trihydrochloride (H₅tacidpdpCl₃)

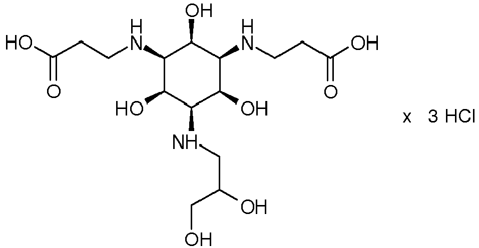

tbcadpn·HCOOH (200 mg, ~ 0.3 mmol) was dissolved in methanol (25 mL). Acetic acid (75 µL), D,L-glyceraldehyde dimer (65 mg, 0.4 mmol) and 5-ethyl-2-methylpyridine borane (80 µL, 0.5 mmol) were successively added. The suspension, which turned into a clear solution within few hours, was stirred for 3 days at rt. The solvent was removed and the residue purified via preparative HPLC (C18 column, solvent: water + 0.1 wt% formic acid (*A*) / acetonitrile (*B*); gradient: from 15 % *B* to 55 % *B* in 15 minutes; UV detection at 235 nm). The combined product fractions were lyophilized. The residue (70 mg) was suspended in 6 M hydrochloric acid (100 mL), heated to reflux for 4 hours and stirred for 12 hours at ambient temperature afterwards. The solution was evaporated to dryness. The remaining solid was dissolved in 0.5 M hydrochloric acid (5 mL) and sorbed on DOWEX 50. The column was washed with water (0.5 L) and 0.5 M hydrochloric acid (0.75 L) and the product was eluted with 3 M hydrochloric acid (1.5 L). The eluant was removed and the solid dried in vacuo to yield 91 mg of H₂tacidpdp·3HCl·xH₂O.

**¹H-NMR** (400 MHz, DMSO-d6, pH* = 0): δ = 2.99 (t, 4H), 3.31 (dd, 1 H), 3.50 (dd, 1 H), 3.57 (t, 4H), 3.67 (dd, 1 H), 3.72 (dd, 1 H), 3.78 (m, 3H), 4.17 (m, 1 H), 4.78 (m, 3H) ppm.

### Example 4b

### Bis[µ₃-3,3'-({[1R(1α,2α,3α,4α,5α,6α)]-2,4,6-trihydroxylato-1 κ²O²O⁶,2κ²O²O⁴,3κ²O⁴O⁶-5-[(3-hydroxy-2-hydroxy)ato-3κO-propyl)amino-3κN⁵]cyclohexane-1,3-diyl}diimino-1 κN¹,2κN³) dipropanoato-1κO,2κO']trihafnium(IV) [Hh₃(H-₃tacidpdp)₂]

H₂tacidpdp·3HCl·xH₂O (85 mg, ~ 0.1 mmol) was dissolved in water (6 mL) and hafnium(IV)tetrachloride (57 mg, 0.2 mmol) dissolved in a small amount of water (2 mL) was added. The pH was adjusted to ~ 4.5 (1 M solution of sodium hydroxide) and the solution was irradiated 45 minutes at 140 °C in the microwave. The solution was desalted via ultrafiltration (cellulose acetate membrane, lowest NMWL 500 g/mol, Millipore). The retentate was again passed through an ultrafiltration cell (cellulose acetate membrane, lowest NMWL 3000 g/mol, Millipore). The filtrate was evaporated to dryness and the white solid dried in vacuo to yield 57 mg of the title compound [Hf₃(H₋₃tacidpd)₂].

MS(ES⁺): *mlz* (%) 657 (100) f[Hf₃(H-₃tacidpdp)₂]+2H}²⁺, 1316 (12) {[Hf₃ (H₋₃tacidpdp)₂]+H}⁺.
MS (ES-): *mlz* (%) 1359.4 (100) {[Hh(H₋₃tacidpdph)₂]+HCOO}⁻.

### Example 5 [Hf₃(H-₄tacidpery)₂]

### Bis[µ₃-3,3'-({[1R(1α,2α,3α,4α,5α,6α)]-5-[(2S,3R)(3,4-dihydroxy-2-hydroxylato-3κO-butyl)amino-3κN⁵]-2,4,6-trihydroxylato-1κ²O²O⁶,2κ²O²O⁴,3κ²O⁴O⁶-cyclohexane-1,3-diyl}diimino-1κN¹,2κN³)dipropanoato-1 κ,2κO']trihafnium(IV)

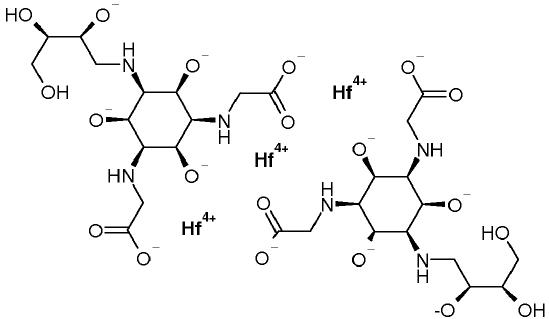

### Example 5a

### 3,3'-([[1R(1α,2α,3α,4α,5α,6α)]-2,4,6-Trihydroxy-5-[(2S,3R)(2,3,4-trihydroxybutyl)-amino]cyclohexane-1,3-diyl}diimino)dipropanoic acid trihydrochloride (H₅tacidperyCl₃)

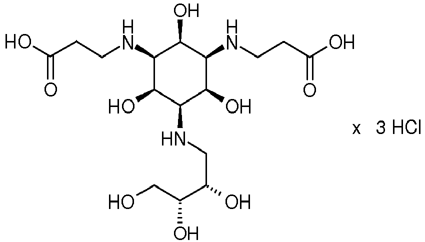

tbcadpn·HCOOH (200 mg, ~ 0.3 mmol) was dissolved in methanol (25 mL). Acetic acid (75 µL), D-erythrose (125 mg, 0.7 mmol) and 5-ethyl-2-methylpyridine borane (80 µL,0.5 mmol) were successively added. The clear solution was stirred for 1 day at ambient temperature. The solvent was removed and the residue purified via preparative HPLC (C18 column, solvent: water + 0.1 wt% formic acid (A) / acetonitrile (*B*); gradient: from 15 % *B* to 55 % *B* in 15 minutes; UV detection at 236 nm). The combined product fractions were lyophilized. The residue (90 mg) was suspended in 6 M hydrochloric acid (100 mL) and heated to reflux for 4 h. The solution was evaporated to dryness. The remaining solid was dissolved in 0.5 M hydrochloric acid (5 mL) and sorbed on DOWEX 50. The column was washed with water (0.5 L) and 0.5 M hydrochloric acid (0.75 L) and the product was eluted with 3 M hydrochloric acid (1.5 L). The eluant was removed and the solid dried in vacuo to yield 70 mg of H₂tacidpery·3HCl·xH₂O.

**¹H-NMR** (300 MHz, D₂O, pH*=0): δ = 2.99 (t, 4H), 3.35 (dd, 1 H), 3.57 (t, 4H), 3.63 (m, 1 H), 3.69 (m, 1 H), 3.75 - 3.82 (m, 5H), 4.10 (m, 1 H), 4.78 (m, 3H) ppm.

### Example 5b

### Bis[µ₃-3,3'-({[1R(1α,2α,3α,4α,5α,6α)]-5-[(2S,3R)(3,4-dihydroxy-2-hydroxylato-3κO-butyl)amino-3κN⁵]-2,4,6-trihydroxylato-1κ²O²O⁶,2κ²O²O⁴,3κ²O⁴O⁶-cyclohexane-1,3-diyl}diimino-1κN¹,2κN³)dipropanoato-1κO,2κO']trihafnium (IV) [Hf₃(H-₃tacidpery)₂]

H₂tacidpery·3HClxH₂O (60 mg, ~ 75 µmol) was dissolved in water (6 mL) and hafnium(IV)tetrachloride (38 mg, 0.1 mmol) dissolved in a small amount of water (2 mL) was added. The pH was adjusted to ~ 4.5 (1 M solution of sodium hydroxide) and the solution was heated 45 minutes at 140 °C in the microwave. The solution was desalted via ultrafiltration (cellulose acetate membrane, lowest NMWL 500 g/mol, Millipore). The retentate was again passed through an ultrafiltration cell (cellulose acetate membrane, lowest NMWL 3000 g/mol, Millipore). The filtrate was evaporated to dryness and the white solid dried in vacuo to yield 40 mg of the title compound [Hf₃(H-₃tacidpery)₂].

**MS (ES⁺):** *m*/*z* (%) 688 (100) ([Hh₃(H₋₃tacidpery)₂]+2H}²⁺, 1375 (31) {[Hf₃ (H-₃tacidpery)₂]+H}⁺.
**MS (ES-):** *mlz* (%) 1419.2 (100) {[Hf₃(H₋₃tacidpery)₂]+HCOO}⁻, 1373.2 (4) {[Hf₃ (H₋₃tacidpery)₂]-H}⁻.

### Example 6 [Hf₃(H₋₄tacidaery)₂]

### Bis[µ₃-2,2'-({[1R(1α,2α,3α,4α,5α,6α)]-5-[(2S,3R)(3,4-dihydroxy-2-hydroxylato-3κO-butyl)amino-3κN⁵]-2,4,6-trihydroxylato-1κ²O²O⁶,2κ²O²O⁴,3κ²O⁴O⁶-cyclohexane-1,3-diyl}diimino-1κN¹,2κN³)diacetato-1κO,2κO']trihafnium(IV)

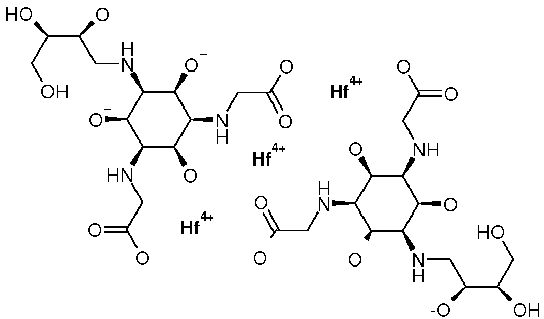

### Example 6a

### Di-tert butyl 2,2'-({[1R(1α,2α,3α,4α,5α,6α)]-2,4,6-tris(benzyloxy)-5-[(2S,3R)(2,3,4-trihydroxybutylyl)amino]cyclohexane-1,3-diyl}diimino)diacetate

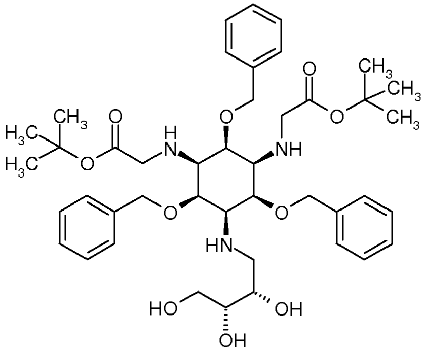

tbcada (2.0 g, 2.96 mmol) was dissolved in methanol (180 mL). Acetic acid (920 µL), D-erythrose (950 mg, 5.92 mmol) and 5-ethyl-2-methylpyridine borane complex (660 µL,4.4 mmol) were successively added. The solution was stirred for 3 days while 5-ethyl-2-methylpyridine borane complex (220 µL,1.5 mmol) and D-erythrose (240 mg, 2.0 mmol) were added additionally after 4 hours. The solvent was removed and the residue was purified by chromatography on amino phase silica gel (ethyl acetate in hexane, 50 to 100%) to yield 1.55 g of the title compound.

**¹H-NMR** (300 MHz, CDCl₃): δ = 1.44 (s, 18H), 2.98 (m, 2H), 3.05 - 3.19 (m, 3H), 3.25 - 3.35 (m , 2H), 3.45 - 3.52 (m, 3H), 3.54 - 3.80 (m, 6H), 4.51 - 4.70 (m, 6H), 7.28 - 7.44 (m, 15H) ppm.

### Example 6b

### 2,2'-({[1R(1α,2α,3α,4α,5α,6α)]-2,4,6-Trihydroxy-5-[(2S,3R)(2,3,4-trihydroxybutyl)-amino]cyclohexane-1,3-diyl}diimino)diacetic acid trihydrochloride (H₅tacidaeryCl₃)

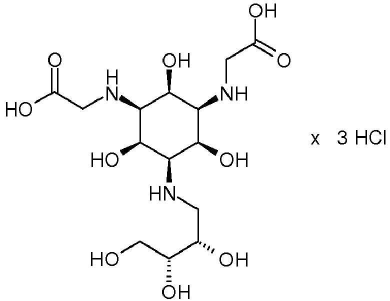

Di-*tert*-butyl 2,2'-({[1*R*-(1α,2α,3α,4α,5α,6α)]-2,4,6-tris[benzyloxy]-5-[(2*S*,3*R*)(2,3,4-tri-hydroxybutylyl)amino]cyclohexane-1,3-diyl]diimino)diacetate (272 mg, 0.35 mmol) was suspended in hydrochloric acid (6 M, 60 mL) and heated to reflux for 4 h. After cooling, the solution was evaporated to dryness, the remaining solid dissolved in 0.5 M hydrochloric acid (5 mL) and sorbed on DOWEX 50. The column was washed with water (0.5 L) and 0.5 M hydrochloric acid (1 L) and the product was eluted with 3 M hydrochloric acid (1.5 L). The eluant was removed and the solid dried in vacuum to yield 172 mg of the title compound H₂tacidaery·3HCl·3.5H₂O.

**¹H-NMR** (400 MHz, D₂O, pH*=0): δ = 3.35 (dd, 1 H), 3.62 (dd, 1 H), 3.68 (m, 1 H), 3.76 - 3.80 (m, 3H), 3.86 (m, 2H), 4.10 (m, 1 H), 4.24 (br., 4H), 4.79 (m, 3H) ppm.
**¹³C-NMR** (101 Mhz, D₂O, pH*=0) δ = 47.7, 50.2, 59.3, 59.4, 64.9, 65.8, 65.87, 65.94, 69.7, 76.0, 171.0 ppm.
**Anal.** Calcd (%) for C₁₄H₂₇N₃O₁₀·3HCl·3.5H₂O (569.82): C, 29.51; H, 6.55; N, 7.37. Found: C, 29.46; H, 6.47; N, 7.36.

### Example 6c

### Bis[µ3-2,2'-({[1R(1α,2α,3α,4α,5α,6α)]-5-[(2S,3R)(3,4-dihydroxy-2-hydroxylato-3κO-butyl)amino-3κN⁵]-2,4,6-trihydroxylato-1κ²O²O⁶,2κ²O⁴,3κ²O⁴O⁶-cyclohexane-1,3-diyl}diimino-1κN¹,2κN³)diacetato-1κO,2κO']trihafnium(IV) [Hf₃(H₋₄tacidaeryl)₂]

A solution of H₂tacidaery·3HCl·3.5H₂O (980 mg, 1.72 mmol) and hafnium(IV)chloride (895 mg, 2.79 mmol) in water (100 mL) was separated into 4 pressure vessels. The pH of each vessel was adjusted to 2.2 by addition of aqueous ammonia (33%) and water was added to reach a total volume of 30 mL. The vessels were sealed and irradiated in a microwave reactor for 45 minutes at 140°C. After addition of hafnium(IV)chloride (4 x 6.5 mg) to each reacion vessel the pH was adjusted to 7.0 by addition of aqueous ammonia (33%) and irradiation in a microwave reactor at 140°C was continued for three hours. The reaction mixtures were combined and the turbid reaction mixture was filtrated and an ultrafiltration of the still turbid filtrate through a 500 da membrane was repeated 3 times by addition of desalted water (3 x 250 mL). The retentate was collected, diluted to a total volume of 300 mL and passed through a 3000 da ultrafiltration membrane while dilution of the retentate was repeated three times. The combined 3000 da filtrates were concentrated in vacuum while a final volume of 100 mL was lyophilized to yield 528 mg of the title compound.

**MS** (ES⁺): *m*/*z=* 1319.0 {[Hf₃(H₋₄tacidaery)₂]+H}⁺.
**MS** (ES-): *m*/*z* = 1316.4 {[Hf₃(H₋₄tacidaery)₂]-H}⁻.

### Example 7 [Hf₃(H-₃tacidpma)₂]

### Bis[µ₃-3,3'-({[1R(1α,2α,3α,4α,5α,6α)]-5-[(carboxylato-3κO-methyl)amino-3κN⁵]-2,4,6-trihydroxylato-1κ²O²O⁶,2κ²O²O⁴,3κ²O⁴O⁶-cyclohexane-1,3-diyl}diimino-1κN¹,2κN³)di-propanoato-1κO,2κO']trihafnium(IV)

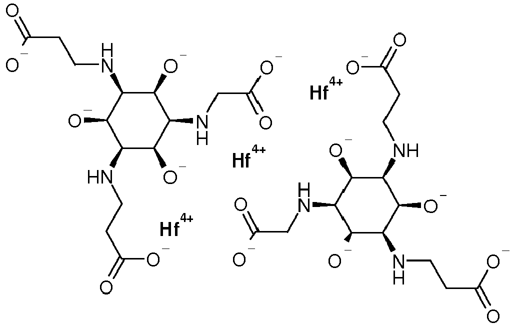

### Example 7a

### 3,3'-({[1R-(1α,2α,3α,4α,5α,6α)]-5-[(Carboxymethyl)amino]-2,4,6-trihydroxycyclohexane-1,3-diyl}diimino)dipropanoic acid trihydrochloride (H₆tacidpmaCl₃)

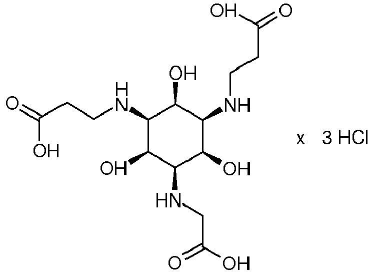

tbcama (300 mg, 0.5 mmol) was dissolved in methanol (30 mL). Acrylonitrile (350 µL, 5.3 mmol) was added and the solution was stirred for 2 days at ambient temperature. The solvent was removed and *tert*-butyl *N*-({[1*R*-(1α,2α,3α,4α,5α,6α)]-2,4,6-tris[benzyloxy]-3,5-bis[(2-cyanoethyl)amino]-cyclohexane-1-yl]imino)acetate was obtained as crude product. The residue (350 mg) was suspended in 6 M hydrochloric acid (50 mL) and heated to reflux for 3 h. After cooling, the solution was evaporated to dryness, the remaining solid dissolved in 0.5 M hydrochloric acid (5 mL) and sorbed on DOWEX 50. The column was washed with water (0.5 L) and 0.5 M hydrochloric acid (1 L) and the product was eluted with 3 M hydrochloric acid (1.2 L). The eluant was removed and the solid dried in vacuo to yield 246 mg of H₃tacidpma·3HCl·2.5H₂O.

**¹H-NMR** (400 MHz, D₂O, pH*= 0): δ = 2.99 (t, 4H), 3.57 (t, 4H), 3.77 (t, 2H), 3.84 (t, 1 H), 4.23 (s, 2H), 4.77 (m, 3H) ppm.
**¹³C-NMR** (101 MHz, D₂O, pH* = 0): δ = 32.9, 43.7, 47.7, 59.6, 59.7, 66.1, 66.2, 171.0, 176.9 ppm.
**Anal.** Calcd (%) for C₁₄H₂₅N₃O₉·3HCl·2.5H₂O (533.78): C, 31.50; H, 6.23; N, 7.87. Found: C, 31.72; H, 6.06; N, 7.90.

### Example 7b

### Bis[µ₃-3,3'-({[1R(1α,2α,3α,4α,5α,6α)]-5-[(carboxylato-3κO-methyl)amino-3κN⁵]-2,4,6-trihydroxylato-1 κ²O²O⁶,2κ²O²O⁴,3κ²O⁴O⁶-cyclohexane-1,3-diyl}diimino-1κN¹,2κN³)di-propanoato-1κO,2κO']trihafnium (IV) [Hh(H₋₃tacidpma)₂]

A solution of 3 H₃tacidpma·3HCl·2.5H₂O (2.66 g, 4.98 mmol) and hafnium(IV)chloride (2.96 g, 9.25 mmol) in water (140 mL) was separated into 14 pressure vessels. The pH of each vessel was adjusted to 1.6 by addition of aqueous ammonia (33%) and water was added to reach a total volume of 30 mL. The vessels were sealed and irradiated in a microwave reactor for 120 minutes at 140°C. The reaction mixtures were combined and the pH of the solution was adjusted to 6.5 by addition of aqueous ammonia (33%). The turbid reaction mixture was filtrated and an ultrafiltration of the still turbid filtrate through a 500 da membrane was repeated 3 times by addition of desalted water. The retentate was collected, diluted to a total volume of 1200 mL and passed through a 3000 da ultrafiltration membrane while dilution of the retentate was repeated two times. The combined 3000 da filtrates were concentrated in vacuum to a final volume of 200 mL and lyophilized to yield 2.62 g of the title compound [Hf₃(H₋₃tacidpma)₂].

**¹H-NMR** (600 MHz, D₂O): δ = 2.47 - 2.67 (m, 8H), 3.08 - 3.21 (m, 4H), 3.22 - 3.34 (m, 4H), 3.46 - 3.58 (m, 4H), 3.59 - 3.76 (m, 3H), 3.80 - 3.96 (m, 2H), 4.01 - 4.21 (m, 2H), 4.78 - 4.98 (m, 6H), 5.00 - 5.13 (m, 2H), 5.13 - 5.22 (m, 1 H), 5.24 - 5.44 (m, 1 H), 5.65 (br., 1 H) ppm.
**MS** (ES⁺): *m*/*z* (%) = 642 (100) {[Hf₃(H₋₃tacidpma)₂]+2H]²⁺, 1283 (24) {[Hf₃ (H₋₃tacidpma)₂]+H}⁺.
**Anal.** Calcd (%) for C₂₈H₃₈Hf₃NeO₁₈·9H₂O (1444.24): C, 23.29; H, 3.91; N, 5.82; Hf, 37.08. Found: C, 23.30; H, 3.92; N, 5.79; Hf, 36.77.

### Example 8 [Hf₃(H₋₄tacidahe)₂]

### Bis[µs-2,2'-({[1R(1α,2α,3α,4α,5α,6α)]-2,4,6-trihydroxylato-1κ²O²O⁶,2κ²O²O⁴,3κ²O⁴O⁶-5-[(2-hydroxylato-3κO-ethyl)amino-3κN⁵]cyclohexane-1,3-diyl}diimino-1κN¹,2κN³)diacetato-1κO,2κO']trihafnium (IV)

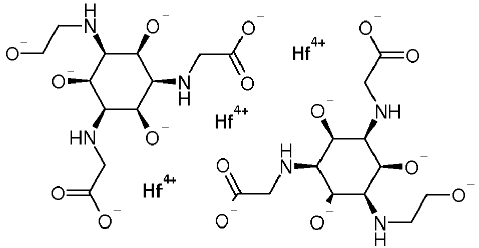

### Example 8a

### Di-tert-butyl 2,2'-({[1R(1α,2α,3α,4α,5α,6α)]-2,4,6-tris[benzyloxy]-5-[(2-hydroxyethyl)-amino]cyclohexane-1,3-diyl}diimino)diacetate

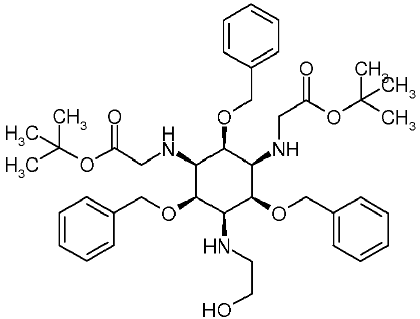

tbcada (2.0 g, 2.96 mmol) was dissolved in methanol (200 mL). Acetic acid (0.6 mL), glycolaldehyde dimer (355 mg, 2.96 mmol) and 5-ethyl-2-methylpyridine borane (660 µL, 4.44 mmol) were successively added. The solution was stirred for 3 days at ambient temperature. The solvent was removed and the residue was purified by chromatography on amino phase silica gel (ethyl acetate in hexane, 50 to 100%) to yield 1.15 g of the title compound.

**¹H-NMR** (400 MHz, DMSO-*d6*): δ = 1.37 (s, 18H), 2.82 (t, 2H), 3.29 (m, 2H), 3.36 - 3.42 (m, 4H), 3.44 - 4.48 (m, 6H), 4.60 (s, 6H), 7.20 - 7.42 (m, 15H) ppm.
**¹³C-NMR** (101 MHz, DMSO-*d6*): δ = 27.7, 52.7, 53.1, 57.8, 58.8, 60.7, 69.6, 69.7, 77.5, 77.7, 79.6, 127.0, 127.0, 127.2, 128.1, 128.2, 138.9, 171.6 ppm.

### Example 8b

### 2,2'-({[1R-(1α,2α,3α,4α,5α,6α)]-2,4,6-Trihydroxy-5-[(2-hydroxyethyl) amino]cyclohexane-1,3-diyl}diimino)diacetic acid trihydrochloride (H₅tacidaheCl₃)

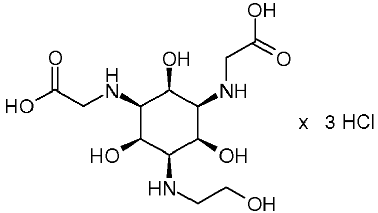

Di-*tert*-butyl 2,2'-({[1*R*-(1α,2α,3α,4α,5α,6α)]-2,4,6-tris[benzyloxy]-5-[(2-hydroxyethyl)-amino]cyclohexane-1,3-diyl}diimino)diacetate (1.49 g, 2.07 mmol) was suspended in hydrochloric acid (6 M, 150 mL) and heated to reflux for 2 h. After cooling, the solution was evaporated to dryness, the remaining solid dissolved in 0.5 M hydrochloric acid (50 mL) and sorbed on DOWEX 50. The column was washed with water (0.5 L) and 0.5 M hydrochloric acid (1 L) and the product was eluted with 3 M hydrochloric acid (1.5 L). The eluant was removed and the solid dried in vacuo to yield 930 mg of the title compound H₂tacidahe·3HCl·3.5H₂O.

**¹H-NMR** (400 MHz, D₂O, pH =0): δ = 3.44 (m, 2H), 3.78 (t, 1 H), 3.85 (t, 2H), 3.98 (m, 2H), 4.23 (br, 4H), 4.77 (t, 3H) ppm.
**¹³C-NMR** (101 MHz, D₂O, pH* = 0): δ = 47.7, 49.7, 59.2, 59.4, 59.6, 66.2, 66.3, 171.0. **Anal.** Calcd (%) for C₁₂H₂₃N₃O₈·3HCl·3.5H₂O (509.76): C, 28.27; H, 6.53; N, 8.24. Found: C, 28.19; H, 6.03; N, 8.26.

### Example 8 c

### Bis[µs-2,2'-({[1R(1α,2α,3α,4α,5α,6α)]-2,4,6-trihydroxylato-1κ²O²O⁶,2κ²O²O⁴,3κ²O⁴O⁶-5-[(2-hydroxylato-3κO-ethyl)amino-3κN⁵]cyclohexane-1,3-diyl}diimino-1κN¹,2κN³)-diacetato-1κo,2κO']trihafnium(IV) [Hf₃(H₋₄tacidahe)₂]

A solution of H₂tacidahe·3HCl·3.5H₂O (0.79 g, 1.68 mmol) and hafnium(IV)chloride (0.82 g, 2.56 mmol) in water (50 mL) was separated into 4 pressure vessels. The pH of each vessel was adjusted to 5.5 by addition of aqueous ammonia (33%) and water was added to reach a total volume of 30 mL. The vessels were sealed and irradiated in a microwave reactor for 45 minutes at 140°C. After addition of hafnium(IV)chloride (4 x 6 mg) to each reaction vessel the pH was adjusted from 2.5 to 7.5 by addition of aqueous ammonia (33%) and irradiation in a microwave reactor at 140°C was continued for three hours. The reaction mixtures were combined and the turbid reaction mixture was filtrated and an ultrafiltration of the filtrate through a 500 da membrane was repeated 3 times by addition of desalted water. The retentate was collected, diluted to a total volume of 1000 mL and passed through a 3000 da ultrafiltration membrane while dilution of the retentate was repeated two times. The combined 3000 da filtrates were concentrated in vacuum while a final volume of 200 mL was lyophilized and yielded 0.81 g of the title compound [Hf₃(H₋₄tacidahe)₂].

**MS** (ES⁺): *m*/*z* 600 {[Hf₃(H₋₃tacidahe)₂]+2H}²⁺, 1199 {[Hf₃(H₋₃tacidahe)₂]+H}⁺.
**MS** (ES⁻): *m*/*z* 1197 {[Hf₃(H₋₃tacidahe)₂]-H}⁻.

### Example 9 [Hf₃(H₋₄tacidahp)₂]

### Bis[µs-2,2'-({[1R(1α,2α,3α,4α,5α,6α)]-2,4,6-trihydroxilato-1κ²O²O⁶,2κ²O²O⁴,3κ²O⁴O⁶-5-[(3-hydroxylato-3κO-propyl)amino-3κN⁵]cyclohexane-1,3-diyl}diimino-1κN¹,2κN³)di-acetato-1κO,2κO'] trihafnium(IV)

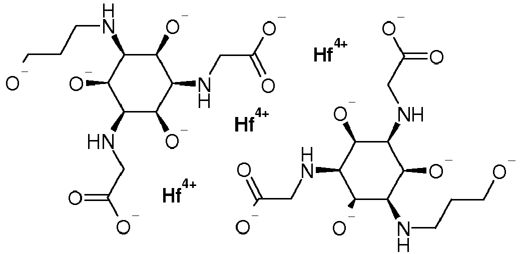

### Example 9a

### Di-tert-butyl 2,2'-({[1R(1α,2α,3α,4α,5α,6α)]-2,4,6-tris[benzyloxy]-5-[(3-hydroxy-propyl)amino]cyclohexane-1,3-diyl}diimino)diacetate

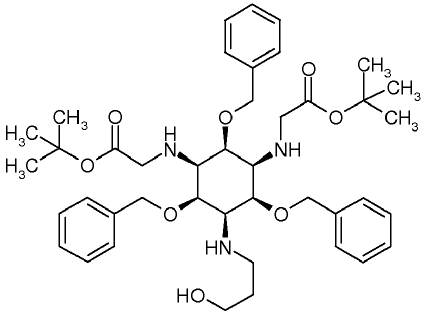

tbcada (308 mg, 0.46 mmol) was dissolved in methanol (11 mL). Acetic acid (89 µL), 3-hydroxypropanal, which was freshly prepared from 3,3-diethoxy-1-propanol (1.0 g, 6.7 mmol) by HCl (10 mL, 0.5 M) treatment at 60°C and isolated via its ether extract, and 5-ethyl-2-methylpyridine borane (92 mg, 0.68 mmol) were successively added. The solution was stirred for 18 hours at ambient temperature. The solvent was removed and the residue the residue was purified by chromatography on amino phase silica gel (ethyl acetate in hexane, 50 to 100%) to yield 256 mg of the title compound.

**¹H-NMR** (300 MHz, DMSO-*d6*): δ = 1.28 - 1.42 (m, 18H), 1.53 (quin, 2H), 2.85 (t, 2H), 3.16 (s, 3H), 3.18 (s, 3H), 3.42 - 3.51 (m, 8H), 4.10 (q, 2H), 4.59 (s, 6H), 7.28 - 7.40 (m, 15H) ppm.

### Example 9b

### 2,2'-({[1R(1α,2α,3α,4α,5α,6α)]-2,4,6-Trihydroxy-5-[(3-hydroxypropyl)amino]cyclohexane-1,3-diyl}diimino)diacetic acid trihydrochloride (H₅tacidahpCl₃)

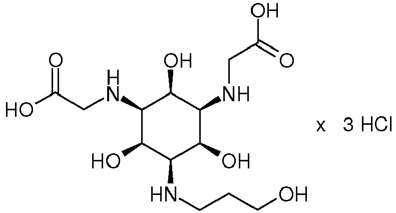

Di-*tert*-butyl 2,2'-({[1*R*-(1α,2α,3α,4α,5α,6α)]-2,4,6-tris[benzyloxy]-5-[(3-hydroxypropyl)-amino]cyclohexane-1,3-diyl}diimino)diacetate (256 mg, 0.35 mmol) was suspended in hydrochloric acid (6 M, 25.6 mL) and heated to reflux for 2 h. After cooling, the solution was evaporated to dryness, the remaining solid dissolved in 0.5 M hydrochloric acid (5 mL) and sorbed on DOWEX 50W-X2. The column was washed with water (200 mL) and 0.5 M hydrochloric acid (250 mL) and the product was eluted with 3 M hydrochloric acid (250 mL). The eluant was removed and the solid solved in methanol (8 mL), Palladium on charcoal (20 mg 10%) was added and the suspension was shaken under a hydrogen athmosphere for 12 hours. The reaction mixture was filtered and dried in vacuo to yield 130 mg of the title compound H₂tacidahp·3HCl.

**¹H-NMR** (400 MHz, D₂O): δ = 2.02 (quin, 2H), 3.34 (t, 2H), 3.65 (t, 1 H), 3.71 - 3.79 (m, 4H), 4.05 (s, 4H), 4.68 (s, 2H), 4.70 (s, 1 H) ppm.

### Example 9 c

### Bis[µs-2,2'-({[1R(1α,2α,3α,4α,5α,6α)]-2,4,6-trihydroxylato-1κ²O²O⁶,2κ²O²O⁴,3κ²O⁴O⁶-5-[(3-hydroxylato-3κO-propyl)amino-3κN⁵]cyclohexane-1,3-diyl}diimino-1κN¹,2κN³)-diacetato-1κO,2κO']trihafnium (IV) [Hf₃(H₋₄tacidahp)₂]

A solution of 2,2'-({[1*R*-(1α,2α,3α,4α,5α,6α)]-2,4,6-trihydroxy-5-[(3-hydroxypropyl)amino]-cyclohexane-1,3-diyl}diimino)diacetic acid trihydrochloride (130 mg, 0.28 mmol) and hafnium(IV)chloride (154 mg, 0.48 mmol) in water (15 mL) was filled into a pressure vessel. The pH was adjusted to 5.5 by addition of aqueous ammonia (33%) and water was added to reach a total volume of 20 mL. The vessel was sealed and irradiated in a microwave reactor for 45 minutes at 140°C. After addition of hafnium(IV)chloride (5 mg) to the reaction vessel the pH was adjusted from 2.5 to 7.5 by addition of aqueous ammonia (33%) and irradiation in a microwave reactor at 140°C was continued for three hours. The reaction mixture was filtrated and an ultrafiltration of the filtrate through a 500 da membrane was repeated 3 times by addition of desalted water (3 x 250 mL). The retentate was collected, diluted to a total volume of 250 mL and passed through a 3000 da ultrafiltration membrane while dilution of the retentate was repeated two times. The combined 3000 da filtrates were concentrated in vacuum while a final volume of 100 mL was lyophilized and yielded 63 mg of the title compound [Hf₃(H₋₄tacidahp)₂].

**MS** (ES⁺): *m*/*z* (%) 1227 (100) {[Hh(H₋₃tacidahp)₂]+H}⁺.
**MS** (ES-): *m*/*z* (%) 1225 (100) {[Hf₃(H₋₃tacidahp)₂]-H}⁻.

### Example 10 [Hf₃(H₋₃tacidamp)₂]

### Bis[µs-2,2'-({[1R(1α,2α,3α,4α,5α,6α)]-5-[(2-carboxylato-3κO-ethyl)amino-3κN⁵]-2,4,6-trihydroxylato-1 κ²O²O⁶,2κ²O²O⁴,3κ²O⁴O⁶-cyclohexane-1,3-diyl}diimino-1κN¹,2κN³)di-acetato-1κO,2κO']trihafnium(IV)

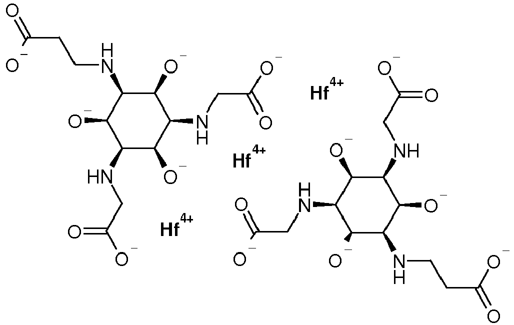

### Example 10a

### Di-tert-butyl 2,2'-({[1R(1α,2α,3α,4α,5α,6α)]-2,4,6-tris[benzyloxy]-5-[(2-cyanoethyl)amino]cyclohexane-1,3-diyl}diimino)diacetate

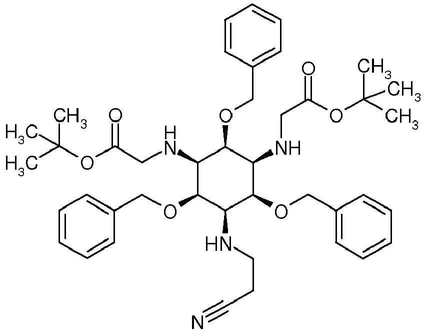

tbcada (500 mg, 0.74 mmol) was dissolved in methanol (50 mL) and acrylonitrile (0.24 mL, 3.7 mmol) was added. The solution was stirred for 24 hours at ambient temperature. The solvent was removed, resolved in methanol, which was removed again. The residue was purified by chromatography on amino phase silica gel (ethyl acetate in hexane, 20 to 100% ) to yield 447 mg of the title compound.

**¹H-NMR** (300 MHz, DMSO-*d6*): δ = 1.32 (s, 18H), 2.92 (t, 2H), 3.28 (m, 6H), 3.42 (s, 6H), 4.48 - 4.61 (m, 6H), 7.21 - 7.39 (m, 15H) ppm.

### Example 10b

### 2,2'-({[1R(1α,2α,3α,4α,5α,6α)]-5-[(2-Carboxyethyl)amino]-2,4,6-trihydroxycyclo-hexane-1,3-diyl}diimino)diacetic acid trihydrochloride (H₅tacidampCl₃)

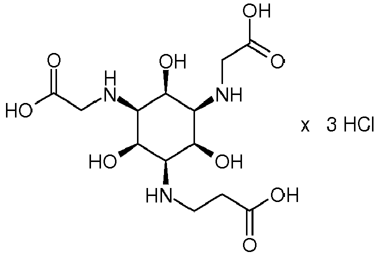

Di-*tert*-butyl 2,2'-({[1*R*-(1α,2α,3α,4α,5α,6α)]-2,4,6-tris[benzyloxy]-5-[(2-cyanoethyl)amino]cyclohexane-1,3-diyl}diimino)diacetate (341 mg, 0.47 mmol) was suspended in hydrochloric acid (6 M, 15 mL) and heated to reflux for 3 h. After cooling, the solution was evaporated to dryness, the remaining solid dissolved in water and sorbed on DOWEX 50W-X2. The column was washed with water (0.5 L) and 0.5 M hydrochloric acid (0.5 L) and the product was eluted with 3 M hydrochloric acid (0.5 L). The eluant was removed and the solid dried in vacuo to yield 170 mg of the title compound H₃tacidamp·3HCl.

**¹H-NMR** (300 MHz, D₂O): δ = 2.88 (t, 2H), 3.46 (t, 2H), 3.62 (t, 1 H), 3.67 (t, 2H), 3.94 (s, 4H), 4.64 (t, 3H) ppm.

### Example 10c

### Bis[µs-2,2'-({[1R(1α,2α,3α,4α,5α,6α)]-5-[(2-carboxylato-3κO-ethyl)amino-3κN⁵]-2,4,6-trihydroxylato-1 κ²O²O⁶,2κ²O²O⁴,3κ²O⁴O⁶-cyclohexane-1,3-diyl}diimino-1κN¹,2κN³)di-acetato-1κO,2κO']trihafnium(IV) [Hf₃(H₋₃tacidamp)₂]

A solution 2,2'-({[1*R*-(1α,2α,3α,4α,5α,6α)]-5-[(2-carboxyethyl)amino]-2,4,6-trihydroxycyclo-hexane-1,3-diylldiimino)diacetic acid trihydrochloride (170 mg, 0.36 mmol) and hafnium(IV)chloride (195 mg, 0.61 mmol) in water (10 mL) was adjusted to a pH of 2.0 by addition of aqueous ammonia (16%) and water was added to reach a total volume of 30 mL. The vessel was sealed and irradiated in a microwave reactor for 120 minutes at 140°C. The solution was adjusted to pH 6.5 by addition of aqueous ammonia (16%). The turbid reaction mixture was filtrated and an ultrafiltration of the still turbid filtrate through a 500 da membrane was repeated 3 times by addition of desalted water. The retentate was collected, diluted to a total volume of 1200 mL and passed through a 3000 da ultrafiltration membrane while dilution of the retentate was repeated two times. The combined 3000 da filtrates were concentrated in vacuum while a final volume of 200 mL was lyophilized and yielded 172 mg of the title compound [Hf₃(H₋₃tacidamp)₂].

**¹H-NMR** (600 MHz, D₂O): δ = 2.49 - 2.70 (m, 4H), 3.10 - 3.21 (m, 2H), 3.22 - 3.34 (m, 2H), 3.46 - 3.63 (m, 2H), 3.64 - 3.79 (m, 4H), 3.83 - 3.96 (m, 4H), 4.03 - 4.23 (m, 4H), 4.78 - 5.10 (m, 6H), 5.20 (br., 1 H), 5.32 - 5.47 (m, 1H), 5.61 - 5.73 (br., 1 H) ppm.
**MS** (ES⁺): *m*/*z* (%) 628 (100) {[Hf₃(H₋₃tacidamp)₂]+2H}²⁺, 1255 (46) {[Hf₃ (H₋₃tacidamp)₂]+H}⁺.

### Example 11 [Hf₃(H₋₄tacidadha)₂]

### Bis[µs-2,2'-({[1R(1α,2α,3α,4α,5α,6α)]-2,4,6-trihydroxylato-1κ²O²O⁶,2κ²O²O⁴,3κ²O⁴O⁶-5-[(1-hydroxy-3-hydroxylato-3κO-propan-2-yl)amino-3κN⁵]cyclohexane-1,3-diyl}diimino-1κN¹,2κN³)diacetato-1κO,2κO']trihafnium (IV)

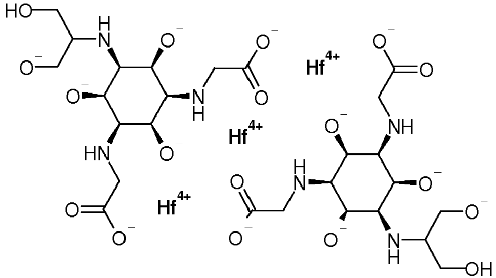

### Example 11a

### Di-tert-butyl 2,2'-({[1R(1α,2α,3α,4α,5α,6α)]-2,4,6-tris[benzyloxy]-5-[(1,3-dihydroxy-propan-2-yl)amino]cyclohexane-1,3-diyl}diimino)diacetate

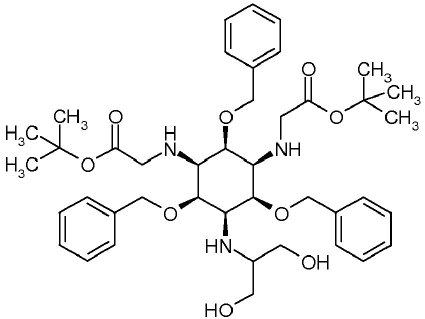

tbcada (1g, 1.48 mmol) was dissolved in methanol (40 mL). Acetic acid (339 µL), dihydroxyacetone (267 mg, 2.96 mmol) and 5-ethyl-2-methylpyridine borane (330 µL, 2.22mmol) were successively added. The reaction was stirred for 3 days at ambient temperature. The solvent was removed and the residue was purified via preparative HPLC (C18 column, solvent: water + 0.1 wt% formic acid (A) / acetonitrile (*B*); gradient: from 40 % *B* to 80 % *B* in 9 minutes; UV detection at 258 nm). The combined product fractions were lyophilized and yielded 560 mg (50%) of the title compound.

**¹H-NMR** (300 MHz, DMSO-*d6*): δ = 1.18 - 1.40 (m, 18 H) 3.06 - 3.24 (m, 1 H) 3.31 - 3.67 (m, 13 H) 4.12 (br. s., 1 H) 4.48 - 4.74 (m, 8 H) 7.15 - 7.46 (m, 15 H) ppm.
**MS** (ESI⁺): *m*/*z* = 791 {M+H}⁺

### Example 11b

### 2,2'-({[1R-(1α,2α,3α,4α,5α,6α)]-2,4,6-Trihydroxy-5-[(1,3-dihydroxypropan-2-yl)amino]-cyclohexane-1,3-diyl}diimino)diacetic acid trihydrochloride (H₂tacidadha)

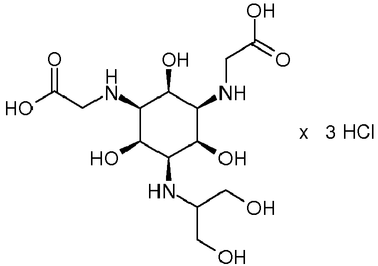

637 mg Di-*tert*-butyl 2,2'-({[1*R*-(1α,2α,3α,4α,5α,6α)]-2,4,6-tris(benzyloxy)-5-[(1,3-di-hydroxypropan-2-yl)amino]cyclohexane-1,3-diyl}diimino)diacetate was suspended in hydrochloric acid (6 M, 70 mL) and heated to reflux for 4 hours and then stirred at ambient temperature for 16 hours. After cooling, the solution was evaporated to dryness, to yield 450 mg (71 %) of the title compound H₂tacidadha·3HCl.

**¹H-NMR** (400 MHz, D₂O, pH* = 0): δ = 3.72 - 3.77 (m, 1 H), 3.87 (t, 2H), 3.92 (dd, 1 H), 3.89-4.02 (m, 3H), 4.24 (s, 4H), 4.76-4.81 (m, 3H) ppm.
**¹³C-NMR** (101 Mhz, D₂O, pH* = 0) δ = 47.8, 57.7, 59.7, 60.8, 61.3, 66.3, 66.5, 171.0 ppm. **MS** (ESI⁺): *m*/*z=* 368 {H₂tacidadha+H}⁺

### Example 11

### Bis[µs-2,2'-({[1R(1α,2α,3α,4α,5α,6α)]-2,4,6-trihydroxylato-1κ²O²O⁶,2κ²O²O⁴,3κ²O⁴O⁶-5-[(1-hydroxy-3-hydroxylato-3κO-propan-2-yl)amino-3κN⁵]cyclohexane-1,3-diyl}diimino-1κN¹,2κN³)diacetato-1κO,2κO']trihafnium(IV) [Hf₃(H₋₃tacidadha)₂]

To a solution of 2,2'-({[1*R*-(1α,2α,3α,4α,5α,6α)]-2,4,6-trihydroxy-5-[(1,3-dihydroxypropan-2-yl)amino]cyclohexane-1,3-diyl}diimino)diacetic acid trihydrochloride (250 mg) in water (10 mL) was added hafnium(IV)chloride (251 mg). The pH was adjusted to 7.3 by addition of aqueous ammonia (33%) and water was added to reach a total volume of 30 mL. The reaction vessel were sealed and irradiated in a microwave reactor for 45 minutes at 140°C and then again for 3h at the same temperature. The turbid reaction mixture was filtrated and an ultrafiltration of the still turbid filtrate through a 500 da membrane was repeated 3 times by addition of desalted water. The retentate was collected, diluted to a total volume of 450 mL and passed through a 3000 da ultrafiltration membrane while dilution of the retentate was repeated two times. The combined 3000 da filtrates were concentrated in vacuum while a final volume of 200 mL was lyophilized and yielded 225 mg (27%) the title compound [Hf₃(H₋₃tacidadha)₂].

**MS** (ESI⁺): *m*/*z=* 1260 {[Hf₃(H₋₃tacidadp)₂]+H}⁺.

### Example 12 [Hf₃(H₋₄tacidaethru)₂]

### Bis[µs-2,2'-({[1R-(1α,2α,3α,4α,5α,6α)]-5-[(3,4-dihydroxy-1-hydroxylato-3κO-butan-2-yl)amino-3κN⁵]-2,4,6-trihydroxylato-1κ²O²O⁶,2κ²O²O⁴,3κ²O⁴O⁶-cyclohexane-1,3-diyl}-diimino-1κN¹,2κN³)diacetato-1 κO,2κO']trihafnium(IV)

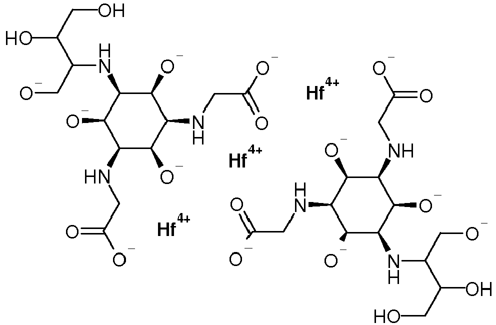

### Example 12 a

### Di-tert-butyl 2,2'-({[1R(1α,2α,3α,4α,5α,6α)]-2,4,6-tris(benzyloxy)-5-[(1,3,4-trihydroxy-butan-2-yl)amino]cyclohexane-1,3-diyl}diimino)diacetate

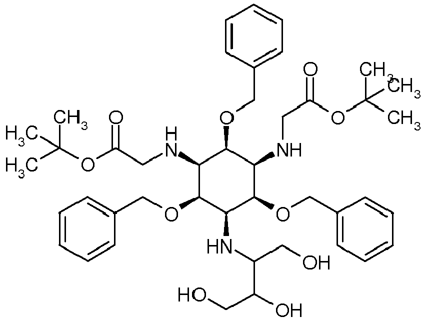

L-(+)Erythrulose (267 mg, 2.2 mmol) was dissolved in methanol/toluene (1:3, 40 mL) and concentrated in vacuo. The residue was re-dissolved in methanol/toluene (1:3, 40 mL) and conccentated in vacuo. To the dried L-(+)erythrulose was added tbcada (500 mg, 0.74 mmol) was dissolved in tetrahydrofuran (20 mL). Acetic acid (111 µL) and 5-ethyl-2-methylpyridine borane (165 µL, 1.11 mmol) were added. The reaction was stirred for 4 days at ambient temperature. The solvent was removed and the residue was purified via preparative HPLC (C18 column, solvent: water + 0.1 wt% formic acid (A) / acetonitrile (*B*); gradient: from 30 % *B* to 70 % *B* in 9 minutes; UV detection at 258 nm). The combined product fractions were lyophilized, to yield 177 mg (31 %) of the title compound.

**¹H-NMR** (300 MHz, DMSO-*d6*): δ = 1.29 - 1.33 (m, 18 H) 2.82 - 2.91 (m, 1 H) 3.32 - 3.60 (m, 18 H) 4.56 - 4.66 (m, 6 H) 7.23 - 7.36 (m, 15 H) ppm.
**MS** (ESI⁺): *m*/*z* = 780 {M+H}⁺.

### Example 12b

### 2,2'-({[1R-(1α,2α,3α,4α,5α,6α)]-2,4,6-Trihydroxy-5-[(1,3,4-trihydroxypropan-2-yl)-amino]cyclohexane-1,3-diyl}diimino)diacetic acid trihydrochloride (H₅tacidaethruCl₃)

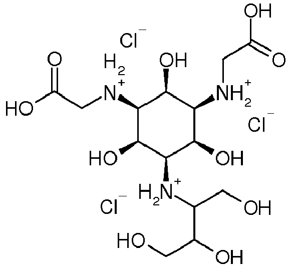

Di-*tert*-butyl 2,2'-({[1*R*-(1α,2α,3α,4α,5α,6α)]-2,4,6-tris(benzyloxy)-5-[(1,3,4-trihydroxy-butan-2-yl)amino]cyclohexane-1,3-diyl}diimino)diacetate (72 mg) was suspended in hydrochloric acid (6 M, 50 mL) and heated to reflux for 4 hours, and then at ambient temperature for 16 hours. The solution was evaporated to dryness, the remaining solid dissolved in water and passed through DOWEX 50X2 resin. The column was washed with water (250 mL) and 0.5 M hydrochloric acid (250 mL) and the product was eluted with 3 M hydrochloric acid (250 mL). The eluent was removed and the solid dried in vacuo, to yield 72 mg of the title compound H₂tacidaethru·3HCl.

**¹H-NMR** (400 MHz, D₂O, pH* = 0): δ = 3,74 (m, 1 H), 3.87 (t, 2H), 3.92 (dd, 2H), 3.98-4.02 (m, 3H), 4.24 (s, 4H), 4.78 (m, 3H) ppm.

**MS** (ESI⁺): *m*/*z=* 398 {[H₂tacidaethru]+H}⁺

### Example 13 [Hf₃(H₋₄tacidahgb)₂]

### Bis[µ₃-2,2'-({[1R-(1α,2α,3α,4α,5α,6α)]-5-[(1-carboxylato-3κO-3-hydroxypropan-1-yl)-amino-3κN⁵]-2,4,6-trihydroxylato-1κ²O²O⁶,2κ²O⁴O⁶,3κ²O⁴O⁶-cyclohexane-1,3-diyl}-diimino-1κN¹,2κN³)diacetato-1 κO,2κO']trihafnium(IV)

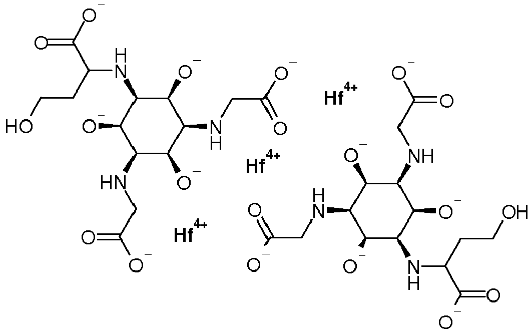

### Example 13 a

### Di-tert-butyl 2,2'-({[1R(1α,2α,3α,4α,5α,6α)]-2,4,6-tris[benzyloxy]-5-[(2-oxotetrahydrofuran-3-yl)amino]cyclohexane-1,3-diyl}diimino)diacetate

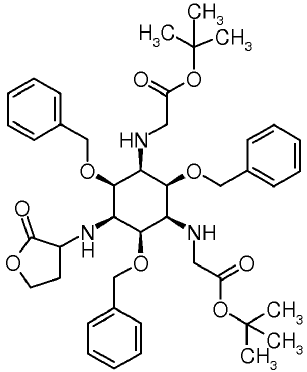

tbcada (1.0 g, 1.48 mmol) was dissolved in tetrahydrofuran (40 mL) and to this solution was added diisopropylethylamine (389 µl, 2.22 mmol) and α-bromo-γ-butyrolactone (274 µL, 2.96 mmol). The reaction was stirred for 2 days at ambient temperature. The solvent was removed and the residue was purified via preparative HPLC (C18 column, solvent: water + 0.1 wt% formic acid (A) / acetonitrile (*B*); gradient: from 30 % *B* for 4 mins and then a gradient to 70 % *B* in 9 minutes; UV detection at 258 nm). The combined product fractions were lyophilized, to yield 733 mg (65%) of the title compound.

**¹H-NMR** (300 MHz, DMSO-*d6*): δ = 1.36 - 1.37 (m, 18 H) 1.91 - 1.98 (m, 1 H) 3.29 - 3.40 (m, 4 H) 3.60 - 3.61 (m, 2 H) 3.85 - 3.89 (m, 1 H) 4.01 - 4.09 (m, 1 H) 4.25 - 4.29 (dt, 1 H) 4.54 - 4.72 (m, 6 H) 7.28 - 7.43 (m, 15 H) ppm.
**MS** (ESI⁺): *m*/*z* = 760 {M+H}⁺

### Example 13 b

### 2,2'-({[1R(1α,2α,3α,4α,5α,6α)]2,4,6-Trihydroxy-5-[(2-oxotetrahydrofuran-3-yl)amino]cyclohexane-1,3-diyl}diimino)diacetic acid trihydrochloride (H₅tacidablCl₃)

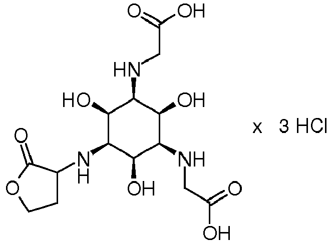

Di-*tert*-butyl 2,2'-({[1*R*-(1α,2α,3α,4α,5α,6α)]-2,4,6-tris[benzyloxy]-5-[(2-oxotetrahydrofuran-3-yl)amino]cyclohexane-1,3-diyl}diimino)diacetate (700 mg, 0.92 mmol) was suspended in hydrochloric acid (6 M, 70 mL) and heated to reflux for 4 h. After cooling, the solution was stirred at ambient temperature for 16 h and then was evaporated to dryness, the remaining solid dissolved in 0.5 M hydrochloric acid (5 mL) and sorbed on DOWEX 50. The column was washed with water (0.5 L) and 0.5 M hydrochloric acid (500 mL) and the product was eluted with 3 M hydrochloric acid (500 mL). The eluent was removed and the solid dried in vacuo to yield 429 mg (96%) of the title compound H₂tacidabl 3HCl.

**¹H-NMR** (400 MHz, D₂O, pH* = 0): δ = 2.56 - 2.71 (m, 1 H), 2.91 - 3.00 (m, 1 H), 3.81 - 3.96 (m,3H), 4.23 (br, 4H), 4.45 - 4.54 (m, 1 H), 3.68 (t, 1 H) 4.76 - 4.84 (m, 4H), ppm. **MS** (ESI⁺) *m*/*z=* 378 {m+H}⁺

### Example 13 c

### Bis[µ3-2,2'-({[1R-(1α,2α,3α,4α,5α,6α)]-5-[(1-carboxylato-3κO-3-hydroxypropan-1-yl)-amino-3κN⁵]-2,4,6-trihydroxylato-1κ²O²O⁴,2κ²O²O⁶,3κ²O²O⁶-cyclohexane-1,3-diyl}diimino-1κN¹,2κN³)diacetato-1κO,2κO']trihafnium(IV) [Hf₃(H₋₄tacidahgb)₂]

To a solution of 2,2'-({[1*R*-(1α,2α,3α,4α,5α,6α)]2,4,6-Trihydroxy-5-[(2-oxotetrahydrofuran-3-yl)amino]cyclohexane-1,3-diyl}diimino)diacetic acid trihydrochloride (100 mg, 0.21 mmol) in water (10 mL) was added hafnium(IV)chloride (224 mg, 0.70 mmol). The pH was adjusted to 7.3 by addition of aqueous ammonia (33%) and water was added to reach a total volume of 30 mL. The reaction vessel was sealed and irradiated in a microwave reactor for 45 minutes at 140°C and then again for 3 h at the same temperature. The turbid reaction mixture was filtrated and an ultrafiltration of the still turbid filtrate through a 500 da membrane was repeated 3 times by addition of desalted water. The retentate was collected, diluted to a total volume of 450 mL and passed through a 3000 da ultrafiltration membrane while dilution of the retentate was repeated two times. The combined 3000 da filtrates were concentrated in vacuum while a final volume of 200 mL was lyophilized and yielded 30mg the title compound [Hf₃(H₋₄tacidahgb)₂].

**MS** (ESI⁺): *m*/*z* = 1336 {[Hf₃(H₋₄tacidahgb)₂]+Na}⁺

### Example 14

### Stability of bis azainositol hafnium complexes

The stability of bis azainositol hafnium complexes was determined in aqueous, buffered solution at pH 7.4. The solution containing 5 mmol/L of the compound in a tightly sealed vessel was heated to 121 °C for 45 minutes in a steam autoclave. The hafnium concentration of the solution was determined by ICP-OES before and after heat treatment. The integrity of the compound was determined by HPLC analysis before and after heat treatment. Absolute stability was calculated as the ratio of the peak area of the compound after and before the heat treatment multiplied with the ratio of the metal concentration of the solution after and before heat treatment.

### HPLC system:

Column 1: Reversed phase C18.
Column 2: ZIC^{®}-HILIC.
Solvent A1: 0,5 mM tetrabutylammonium phosphate pH 6
Solvent A2: 0.1 % formic acid in water
Solvent B1: methanol
Solvent B2: acetonitrile

The use of columns, solvents, flow rate and gradients is detailed in the table below. Detector: element specific detection by ICP-MS, running at m/z 180, the most abundant isotope of hafnium

| | | Chromatographic conditions | | | | |
|---|---|---|---|---|---|---|
| Example No | Stability | Column | Solvent A | Solvent B | Gradient | Flow |
| 1 | 100 % | 1 | A1 | B1 | 0-100% B1 in 10 min | 1 mL/min |
| 2 | 100 % | 2 | A2 | B2 | 50-0% B2 in 10 min | 0.8 mL/min |
| 3 | 100 % | 2 | A2 | B2 | 60-15% B2 in 10 min | 0.8 mL/min |
| 4 | 99 % | 2 | A2 | B2 | 60-15% B2 in 10 min | 0.8 mL/min |
| 5 | 101 % | 2 | A2 | B2 | 60-15% B2 in 10 min | 0.8 mL/min |
| 6 | 101 % | 2 | A2 | B2 | 60-15% B2 in 10 min | 0.8 mL/min |
| 7 | 100 % | 2 | A2 | B2 | 60-15% B2 in 10 min | 0.8 mL/min |
| 8 | 100 % | 2 | A2 | B2 | 60-15% B2 in 10 min | 0.8 mL/min |

## Claims

1. Trinuclear hafnium complexes of general formula (I), 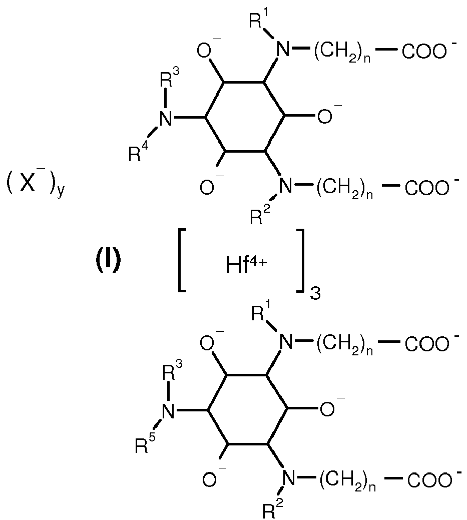 wherein
the substituents at the cyclohexyl ring exhibit an all-*cis* configuration;
R¹, R² and R³ are independently from each other H or CH₃;
R⁴ is H, CH₃, CH₂CH₃, CH₂CH₂CH₃, CH₂CH₂OH CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH, CH(CH₂OH)(CH₂COO⁻), CH(CH₂CH₂OH)(COO⁻), or CH(CH₂OH)(COO⁻);
R⁵ is H, CH₃, CH₂CH₃, CH₂CH₂CH₃, CH₂CH₂OH CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH, CH(CH₂OH)(CH₂COO⁻), CH(CH₂CH₂OH)(COO⁻), or CH(CH₂OH)(COO⁻);
n is 1 or 2;
m is 1 or 2;
y is 0, 1 or 2;
and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

2. The compound according to claim 1,
wherein
the substituents at the cyclohexyl ring exhibit an all-*cis* configuration;
R¹, R² and R³ are independently from each other H or CH₃;
R⁴ is H, CH₂CH₂OH CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH, CH(CH₂OH)(CH₂COO⁻), CH(CH₂CH₂OH)(COO⁻), or CH(CH₂OH)(COO⁻);
R⁵ is H, CH₂CH₂OH CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH, CH(CH₂OH)(CH₂COO⁻), CH(CH₂CH₂OH)(COO⁻), or CH(CH₂OH)(COO⁻);
n is 1 or 2;
m is 1 or 2;
y is 0, 1 or 2;
and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

3. The compound according to claim 1 or 2,
wherein
the substituents at the cyclohexyl ring exhibit an all-*cis* configuration;
R¹, R² and R³ are independently from each other H or CH₃;
R⁴ is CH₂CH₂OH CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH, or CH(CH₂CH₂OH)(COO⁻);
R⁵ is H, CH₂CH₂OH CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH, CH(CH₂OH)(CH₂COO⁻), CH(CH₂CH₂OH)(COO⁻), or CH(CH₂OH)(COO⁻);
n is 1 or 2;
m is 1 or 2;
y is 0, 1 or 2;
and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

4. The compound according to any of the claims 1, 2 or 3, wherein
wherein
the substituents at the cyclohexyl ring exhibit an all-*cis* configuration;
R¹, R² and R³ are independently from each other H or CH₃;
R⁴ is CH₂CH₂OH CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH, or CH(CH₂CH₂OH)(COO⁻);
R⁵ is H, CH₂CH₂OH CH₂CH₂CH₂OH, CH₂CH(OH)CH₂OH, CH₂CH(OH)CH(OH)CH₂OH, CH(CH₂OH)₂, (CH₂)₍₃₋ₙ₎COO⁻, CH(CH₂OH)(CH(OH)CH₂OH, or CH(CH₂CH₂OH)(COO⁻);
n is 1 or 2;
m is 1 or 2;
y is 0, 1 or 2;
and
X⁻ is OH⁻ or Cl⁻;
or a protonated species, a deprotonated species, a regioisomer, a stereoisomer, a tautomer, a hydrate, a solvate, or a pharmaceutically acceptable salt thereof, or a mixture of same.

5. The compound according to any of the claims 1 to 4, which is selected from the group consisting of :
[Hf₃(H₋₃macitp)(H₋₃macidp)OH] = Hydroxido-3κ*O*-[µ₃-3,3',3"-({[1*R*-(1α,2α,3α,4α,5α,6α)]-2,4,6-trihydroxylato-1 κ²*O*²*O*⁶,2κ²*O*²*O*⁴,3κ²*O*⁴*O*⁶-cyclohexane-1,3,5-triyl}tris{methylimino-1κ*N*¹,2κ*N*³,3κ*N*⁵})tripropanoato-1κ*O*,2κ*O*',3κ*O*"] [µ₃-3,3'-({[1*R*-(1α,2α,3α,4α,5α,6α)]-2,4,6-trihydroxylato-1 κ²*O*²*O*⁶,2κ²*O*²*O*⁴,3κ²*O*⁴*O*⁶-5-[methylamino-3κ*N*⁵]cyclohexane-1,3-diyl}bis-{methylimino-1κ*N*¹,2κ*N*³})dipropanoato-1κ*O*,2κ*O*]trihafnium(IV) ;
[Hf₃(H₋₄tacidadhp)₂] = Bis[µ₃-2,2'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[(3-hydroxy-2-hydroxylato-3κ*O*-propyl)amino-3κ*N*⁵]cyclohexane-1,3-diyl}diimino-1κ*N*¹,2κ*N*³)diacetato-1κ*O*,2κ*O'*]trihafnium (IV) ;
[Hf₃(H₋₄tacidphe)₂] = Bis[µs-3,3'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[(2-hydroxylato-3κ*O*-ethyl)amino-3κ*N*⁵]cyclohexane-1,3-diyl}diimino-1κ*N*¹,2κ*N*³)dipropanoato-1κ*O*,2κ*O*']trihafnium(IV) ;
[Hf₃(H₋₄tacidpdhp)₂] = Bis[µs-3,3'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[(3-hydroxy-2-hydroxylato-3κ*O*-propyl)amino-3κ*N*⁵]cyclohexane-1,3-diyl}diimino-1κ*N*¹,2κ*N*³)dipropanoato-1κ*O*,2κ*O*']trihafnium(IV) ;
[Hf₃(H₋₄tacidpery)₂] = Bis[µs-3,3'-({[1*R*-(1α,2α,3α,4α,5α,6α)]-5-[(2*S*,3*R*)(3,4-dihydroxy-2-hydroxylato-3κ*O*-butyl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)dipropanoato-1κ*O*,2κ*O*']trihafnium(IV) ;
[Hf₃(H₋₄tacidaery)₂] = Bis[µs-2,2'-({[1*R*-(1α,2α,3α,4α,5α,6α)]-5-[(2*S*,3*R*)(3,4-dihydroxy-2-hydroxylato-3κ*O*-butyl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)diacetato-1κ*O*,2κ*O'*]trihafnium(IV) ;
[Hf₃(H₋₃tacidpma)₂] = Bis[µ₃-3,3'-({[1*R*-(1α,2α,3α,4α,5α,6α)]-5-[(carboxylato-3κ*O*-methyl)-amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)dipropanoato-1κ*O*,2κ*O*']trihafnium(IV) ;
[Hf₃(H₋₄tacidahe)₂] = Bis[µ₃-2,2'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[(2-hydroxylato-3κ*O*-ethyl)amino-3κ*N*⁵]cyclohexane-1,3-diyl}-diimino-1κ*N*¹,2κ*N*³)diacetato-1κ*O*,2κ*O'*]trihafnium(IV) ;
[Hf₃(H₋₄tacidahp)₂] = Bis[µ₃-2,2'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[(3-hydroxylato-3κ*O*-propyl)amino-3κ*N*]cyclohexane-1,3-diyl}diimino-1κ*N*¹,2κ*N*³)diacetato-1κ*O*,2κ*O*']trihafnium(IV) ;
[Hf₃(H₋₃tacidamp)₂] = Bis[µ₃-2,2'-({[1*R*-(1α,2α,3α,4α,5α,6α)]-5-[(2-carboxylato-3κ*O*-ethyl)-amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1 κ*N*¹,2κ*N*³)diacetato-1κ*O*,2κ*O'*]trihafnium(IV) ;
[Hf₃(H₋₄tacidadha)₂] = Bis[µ₃-2,2'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[(1-hydroxy-3-hydroxylato-3κ*O*-propan-2-yl)amino-3κ*N*⁵]-cyclohexane-1,3-diyl}diimino-1κ*N*¹,2κ*N*³)diacetato-1κ*O*,2κ*O'*]trihafnium(IV) ;
[Hf₃(H₋₄tacidaethru)₂] = Bis[µ₃-2,2'-({[1*R*-(1α,2α,3α,4α,5α,6α)]-5-[(3,4-dihydroxy-1-hydroxylato-3κ*O*-butan-2-yl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)diacetato-1κ*O*,2κ*O'*]trihafnium(IV) ; and
[Hf₃(H₋₄tacidaghb)₂] = Bis[µ₃-2,2'-({[1*R*-(1α,2α,3α,4α,5α,6α)]-5-[(1-carboxylato-3κ*O*-3-hydroxypropan-1-yl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³)diacetato-1κ*O*,2κ*O'*]trihafnium(IV) .

6. Process for the preparation of trinuclear hafnium complexes of the general formula
(I) according to the claim 1, from carboxylic acids of the general formula (II), 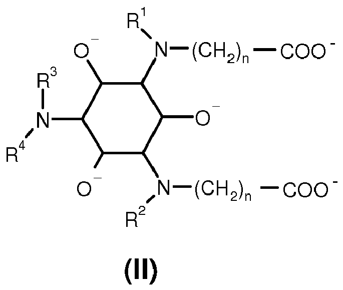 wherein
the substituents at the cyclohexyl ring exhibit an all-*cis* configuration;
R¹, R² and R³ are independently from each H or CH₃;
R⁴ is H, CH₃, CH₂CH₃, CH₂CH₂CH₃, CH₂CH₂OH CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH, CH(CH₂OH)(CH₂COO⁻), CH(CH₂CH₂OH)(COO⁻), or CH(CH₂OH)(COO⁻);
n is 1 or 2; and
m is 1 or 2;
and a metal (IV) halogenide,
wherein
metal is Hafnium;
and
halogenide is either chloride or bromide,
and hydrates thereof,
in aqueous solution under elevated temperatures, using conventional methods or microwave irradiation, ranging from 80°C to 180°C, in a pH range of 1 to 7, preferably at 110 ° to 160 °C in a pH range of 2 to 7.

7. Use of a compound of any one of claims 1 to 5, including any protonated species, any deprotonated species, any stereoisomer, tautomer, hydrate, solvate, or any pharmaceutically acceptable salt thereof, or a mixture of same, for diagnostic imaging.

8. A compound according to any one of claims 1 to 5, including any protonated species, any deprotonated species, any stereoisomer, tautomer, hydrate, solvate, or any pharmaceutically acceptable salt thereof, or a mixture of same, for use in the diagnosis of a disease.

9. Use of a compound of any one of claims 1 to 5, including any protonated species, any deprotonated species, any stereoisomer, tautomer, hydrate, solvate, or any pharmaceutically acceptable salt thereof, or a mixture of same, for the diagnosis of a disease.

10. Use of a compound of any one of claims 1 to 5, including any protonated species, any deprotonated species, any stereoisomer, tautomer, hydrate, solvate, or any pharmaceutically acceptable salt thereof, or a mixture of same, as diagnostic agent.

11. Use of a compound of any one of claims 1 to 5, including any protonated species, any deprotonated species, any stereoisomer, tautomer, hydrate, solvate, or any pharmaceutically acceptable salt thereof, or a mixture of same, as X-ray diagnostic agent.

12. A compound according to any one of claims 1 to 5, including any protonated species, any deprotonated species, any stereoisomer, tautomer, hydrate, solvate, or any pharmaceutically acceptable salt thereof, or a mixture of same, for the manufacture of diagnostic agents.

13. A compound according to any one of claims 1 to 5, including any protonated species, any deprotonated species, any stereoisomer, tautomer, hydrate, solvate, or any pharmaceutically acceptable salt thereof, or a mixture of same, for the manufacture of X-ray diagnostic agents.
